# EUROPEAN PATENT APPLICATION

(11) **EP 1 632 230 A1**
(43) Date of publication of application: **08.03.2006**
(21) Application number: 04736808.9
(22) Date of filing: 14.06.2004
(51) Int. Cl.: A61K 31/445, A61P 25/00, G01N 33/50, C07D 211/32

(54) **NEUROCYTE PROTECTIVE AGENT**

(30) Priority: 12.06.2003 JP 2003167744
(71) Applicant: Eisai Co., Ltd., Tokyo 112-8088 (JP)
(72) Inventor: AKASOFU, Shigeru, Tsukuba Res. Lab. Eisai Co. Ltd, Tsukuba-shi, Ibaraki 3002635 (JP); KIMURA, Manami, Tsukuba Res. Lab. Eisai Co. Ltd, Tsukuba-shi, Ibaraki 3002635 (JP)
(74) Representative: Woods, Geoffrey Corlett
(86) International application number: PCT/JP2004/008669
(87) International publication number: WO 2004/110444

(57) **Abstract**

The present invention relates to a protective agent for neurons of the central nervous system and a prophylactic and/or therapeutic agent for disorders in neurons of the central nervous system, each comprising a compound represented by donepezil hydrochloride.

SEQUENCE LISTING

<110>  Eisai  Co., Ltd.

<120>  Neurocyte protective agent

<130>  N.96293

<140>  EP 04736808.9
<141>  2004-06-14

<150>  PCT/JP04/08669
<151>  2004-06-14

<150>  JP 2003-167744
<151>  2003-06-12

<160>  4

<170>  PatentIn version 3.2

<210>  1
<211>  21
<212>  DNA
<213>  Artificial

<220>
<223>  siRNA

<400>  1
aaugucagug acucuguuut t                                                21

<210>  2
<211>  21
<212>  DNA
<213>  Artificial

<220>
<223>  siRNA

<400>  2
aaacagaguc acugacauut t                                                21

<210>  3
<211>  21
<212>  DNA
<213>  Artificial

<220>
<223>  siRNA

<400>  3
gcugccucaa gaaaguauct t                                                21

<210>  4
<211>  21
<212>  DNA
<213>  Artificial

<220>
<223>  siRNA

<400>  4
gauacuuucu ugaggcagct t                                                21

## Description

### TECHNICAL FIELD

The present invention relates to a neurocyte protective agent comprising donepezil hydrochloride.

### BACKGROUND ART

Donepezil hydrochloride is a substance that activates the cholinergic nervous system in the brain by reversibly inhibiting acetylcholinesterase (an enzyme which degrades acetylcholine) to increase the amount of intracerebral acetylcholine. This substance is widely used as a therapeutic for Alzheimer-type senile dementia and Alzheimer's disease (Japanese Patent No. 2578475), and various research institutes have studied donepezil hydrochloride. Zin Zhou et al. published an article reporting that acetylcholinesterase inhibitor has protective effect on ischemia-like cytotoxicity of rat PC12 cells (tumor cells); in this article, donepezil hydrochloride is used as one example of acetylcholinesterase inhibitor (Zhou, J., Fu, Y and Tang, X. C., 2001. Huperzine A and donepezil protect rat pheochromocytoma cells against oxygen-glucose deprivation. Neurosci. Lett. 306,53-56).

However, details of the protective effect of donepezil hydrochloride on disorders in neurons have not been elucidated yet.

PC 12 cells used in the above-mentioned article are pheochromocytoma cells that are catecholamine-producing tumor derived from pheochromocytes, such as adrenal medulla or sympathetic ganglion cells. Therefore, PC12 cells are not neurons of the brain, and it is known that PC 12 cells are not forming synapse between cells and do not have a function of responding to excitative substances (Sucher, N. J, 1993. Expression of Endogenous NMDAR1 Transcripts without Receptor Protein Suggests Post-transcriptional Control in PC12 Cells. The journal of Biological Chemistry. Vol. 268, No. 30, 22299-22304). That is, the above-described article only examined cancerized cells and did not make any examination using primary culture neurons newly prepared from neurons of the brain. Therefore, no data have been known yet which prove the protective effect of donepezil hydrochloride on ischemia-like disorders in actual neurons.

### DISCLOSURE OF THE INVENTION

The present invention aims at providing drugs which protect neurons (especially, neurons of the central nervous system).

As a result of intensive and extensive researches toward solution of the above problem, the present inventors have found that, surprisingly, donepezil hydrochloride has protective effect on neurons (especially, neurons of the central nervous system). Thus, the present invention has been achieved based on this finding. The present invention is as described below.
(1) A protective agent for neurons of the central nervous system, comprising any one of the compounds shown in the following (i) to (vii) (encompassing the compounds disclosed in Japanese Unexamined Patent Publication No. H1-79151). The salts of these compounds are preferably hydrochloride salts.
   (i) 1-benzyl-4-[(5,6-dimethoxy-1-indanone)-2-yl]methylpiperidine represented by the following chemical formula or a pharmacologically acceptable salt thereof: 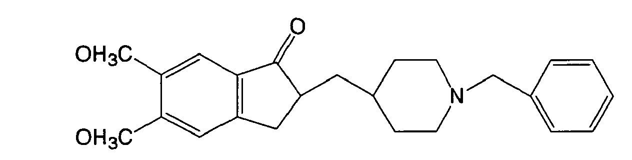 (Japanese Patent No. 2578475, Claim (hereinafter abbreviated to "cl.") 1)
   (ii) a cyclic amine derivative represented by the following general formula (I) or a pharmacologically acceptable salt thereof: 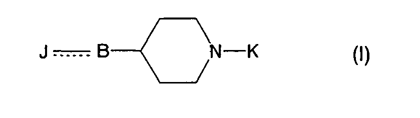 where J is a monovalent or divalent group selected from the groups represented by the following formulas: 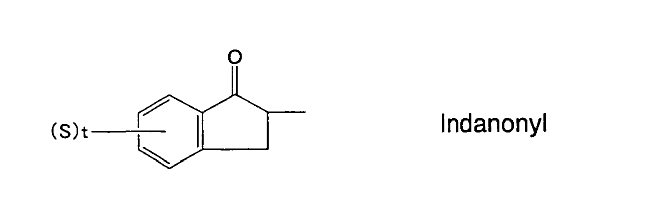 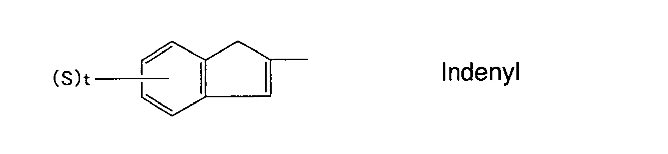 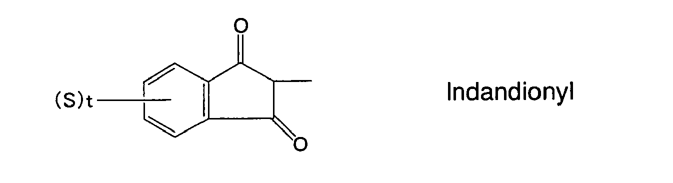 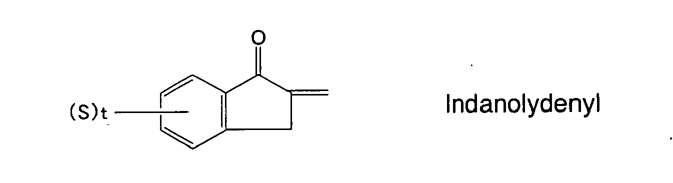 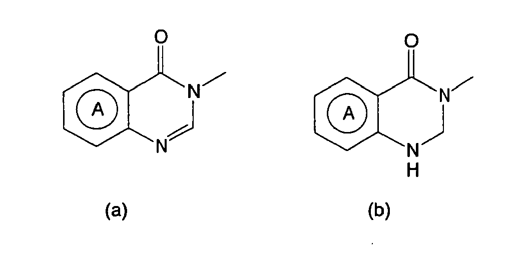 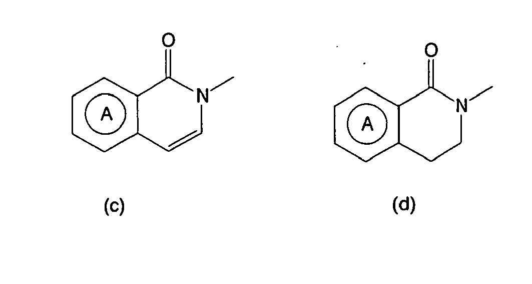 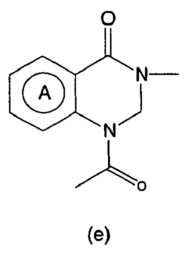 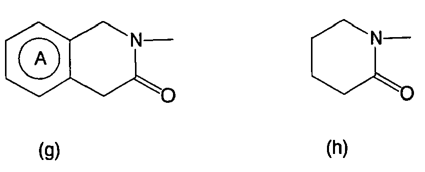 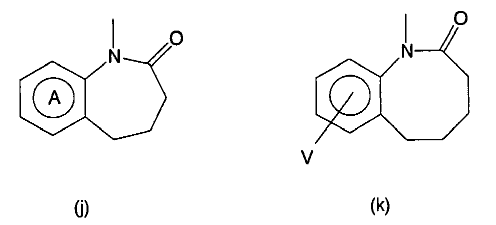 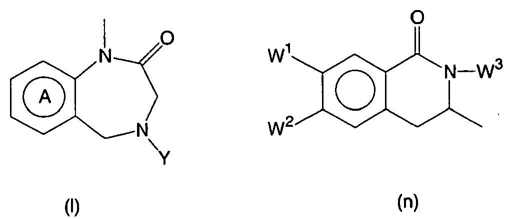 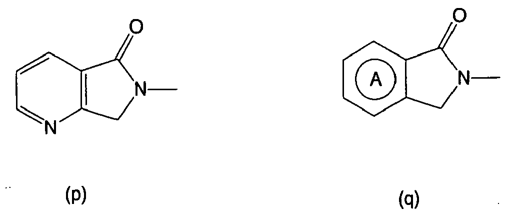 where S is a lower alkyl group with 1 to 6 carbon atoms, a lower alkoxy group with 1 to 6 carbon groups, a halogen atom or a hydroxyl group; t is 0 or an integer from 1 to 4; (S)ₜ may form a methylenedioxy or ethylenedioxy group between adjacent carbon atoms on the phenyl ring linked; Y in formula (1) is a hydrogen atom or a lower alkyl group with 1 to 6 carbon atoms; V in formula (k) is a hydrogen atom or a lower alkoxy group with 1 to 6 carbon atoms; W¹ and W² in formula (n) independently represent, similarly or differently, a hydrogen atom, a lower alkyl group with 1 to 6 carbon atoms, or a lower alkoxy group with 1 to 6 carbon atoms; W³ is a hydrogen atom or a lower alkyl group with 1 to 6 carbon atoms; phenyl ring A in formulas (a) to (e), (g), (j), (1) and (q) may be substituted with an alkyl group with 1 to 6 carbon atoms or a alkoxy group with 1 to 6 carbon atoms;
      B is a group represented by a formula -(CHR²)ₙ- (where n is 0 or an integer from 1 to 10; R² is each independently a hydrogen atom or a methyl group), a group represented by a formula =(CH-CH=CH)_{b}- (where b is an integer from 1 to 3), a group represented by a formula =CH-(CH₂)_{c}- (where c is 0 or an integer from 1 to 9), or a group represented by a formula =(CH-CH)_{d}= (where d is 0 or an integer from 1 to 5);
      K is a phenylalkyl group that may have, as a substituent, an alkyl group with 1 to 6 carbon atoms which may be halogenated, an alkoxy group with 1 to 6 carbon atoms, a nitro group, a halogen atom, a carboxyl group, a benzyloxy group, an alkoxycarbonyl group with 1 to 6 carbon atoms, an amino group, a monoalkylamino group with 1 to 6 carbon atoms, a dialkylamino group with 1 to 6 carbon atoms, a carbamoyl group, an acylamino group with 1 to 6 carbon atoms, a cyclohexyloxycarbonyl group, an alkylaminocarbonyl group with 1 to 6 carbon atoms, an alkylcarbonyloxy group with 1 to 6 carbon atoms, a hydroxyl group, a formyl group or an alkoxy (with 1 to 6 carbon atoms)-alkyl (with 1 to 6 carbon atoms) group; and 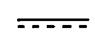 represents a single bond or a double bond;
   (iii) a cyclic amine derivative selected from the compounds represented by the following formulas or a pharmacologically acceptable salt thereof: 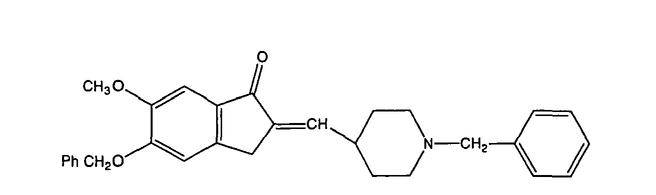 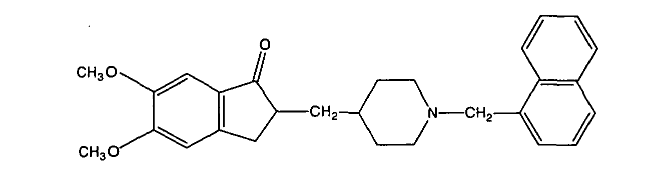 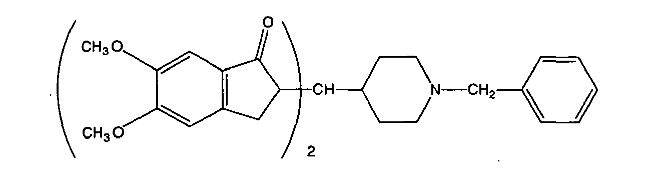 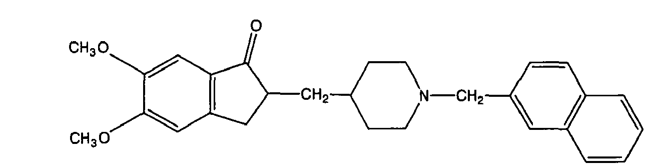 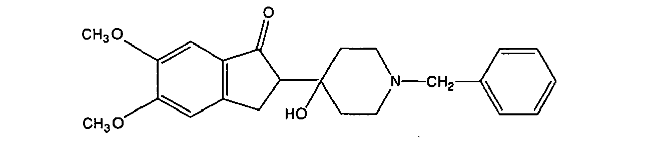 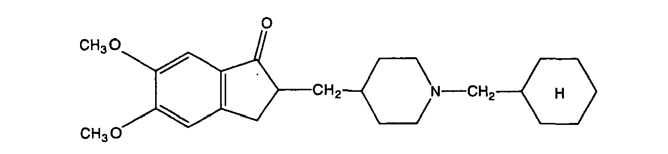 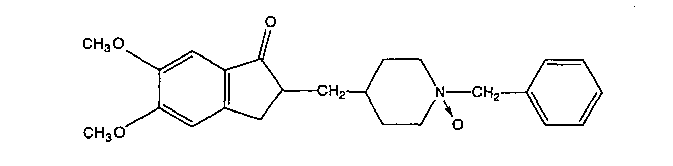 (Japanese Patent No. 2733203, cl. 7)
   (iv) a cyclic amine derivative selected from the compounds represented by the following formulas or a pharmacologically acceptable salt thereof: 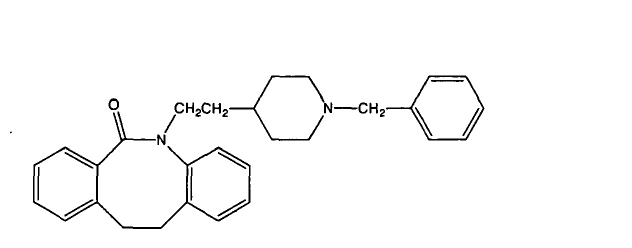 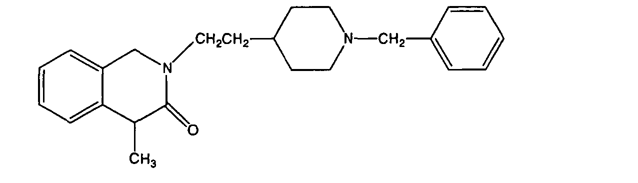 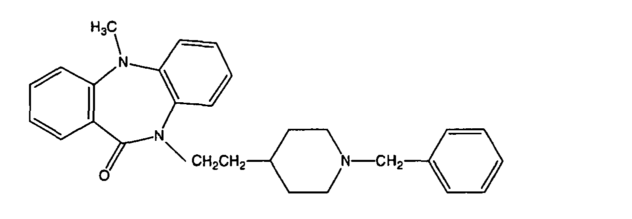 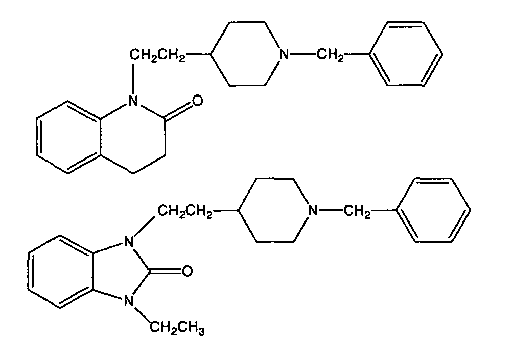 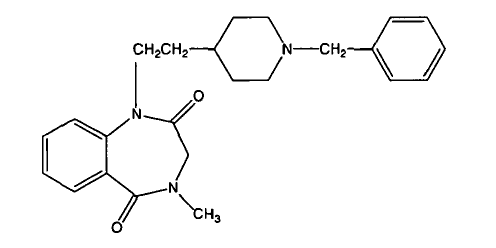 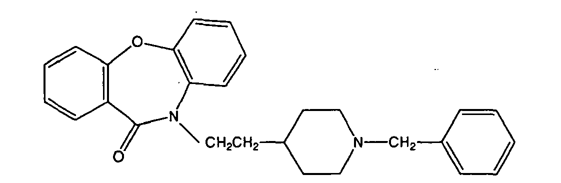 (Japanese Patent No. 2733203, cl. 8)
   (v) a cyclic amine derivative represented by the following general formula (I-1) or a pharmacologically acceptable salt thereof: 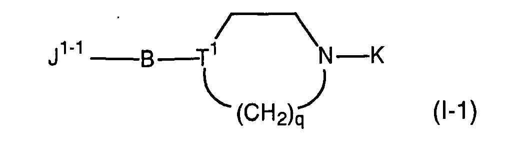 where J¹⁻¹ is a lower alkyl group with 1 to 6 carbon atoms (hereinafter, just referred to as "lower alkyl group"); a cyclohexyl group; a phenyl, pyridyl or pyrazyl group which may have, as a substituent, a lower alkyl group, a lower alkoxy group with 1 to 6 carbon atoms (hereinafter, just referred to as "lower alkoxy group"), a nitro group, a halogen, a carboxyl group, a lower alkoxycarbonyl group, an amino group, a mono-lower alkylamino group, a di-lower alkylamino group, a carbamoyl group, an acylamino group derived from aliphatic saturated monocarboxylic acid with 1 to 6 carbon atoms, a cyclohexyloxycarbonyl group, a lower alkylaminocarbonyl group, a lower alkylcarbonyloxy group, a halogenated lower alkyl group, a hydroxyl group, a formyl group or a lower-alkoxy-lower-alkyl group; a group represented by a formula 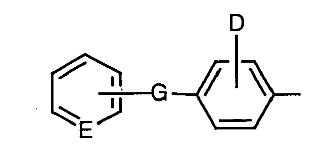 (where G is a group represented by a formula 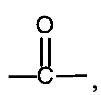 a group represented by a formula 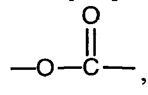 a group represented by a formula -O- , a group represented by a formula 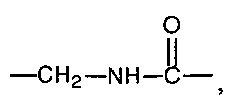 a group represented by a formula -CH₂-O-, a group represented by a formula -CH₂-SO₂- , a group represented by a formula 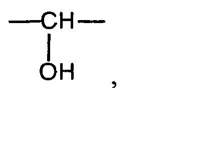 or a group represented by a formula 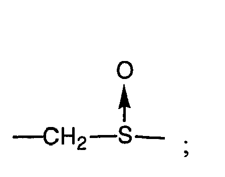 E is a carbon atom or a nitrogen atom);
      a quinolyl group; a quinoxalyl group; a furyl group or a group represented by a formula R¹-CH=CH- (where R¹ is a hydrogen atom or a lower alkoxycarbonyl group); B is a group represented by a formula -(CH₂)ₙ-, a group represented by a formula -NR²-(CH₂)ₙ (where R² is a hydrogen atom, a lower alkyl group, a phenyl group or a lower alkylsulfonyl group), a group represented by a formula -CONR³-(CH₂)ₙ- (where R³ is a hydrogen atom, a lower alkyl group, a phenyl, benzyl or pyridyl group which may have, as a substituent, a lower alkyl group, a lower alkoxy group, a halogen or a hydroxyl group), a group represented by a formula -NH-CO-(CH₂)ₙ-, a group represented by a formula -CH₂-CO-NH-(CH₂)ₙ-, a group represented by a formula -CO-CH₂-CH(OH)-CH₂-, a group represented by a formula -CO-(CH₂)ₙ-, a group represented by a formula -C(OH)-(CH₂)ₙ- or a group represented by a formula -CO-CH=CH-CH₂-; and n in the above formulas is 0 or an integer from 1 to 10; T¹ is a carbon atom;
      K is a phenylalkyl group (where the alkyl has 1 to 2 carbon atoms) in which the phenyl may have, as a substituent, a lower alkyl group, a lower alkoxy group, a nitro group, a halogen, a carboxyl group, a lower alkoxycarbonyl group, an amino group, a mono-lower alkylamino group, a di-lower alkylamino group, a carbamoyl group, an acylamino group derived from aliphatic saturated monocarboxylic acid with 1 to 6 carbon atoms, a cyclohexyloxycarbonyl group, a lower alkylaminocarbonyl group, a lower alkylcarbonyloxy group, a halogenated lower alkyl group, a hydroxyl group, a formyl group or a lower-alkoxy-lower-alkyl group; a cinnamyl group; a lower alkyl group; a pyridyl methyl group; a cycloalkyl (with 3 to 6 carbon atoms)-alkyl group; an adamantanemethyl group; a furfuryl group; a cycloalkyl group with 3 to 6 carbon atoms; or an acyl group; and
      q is 1 or 2; (Japanese Patent No. 3078244, cl. 1)
   (vi) a cyclic amine derivative represented by the following general formula (I-2) or a pharmacologically acceptable salt thereof: 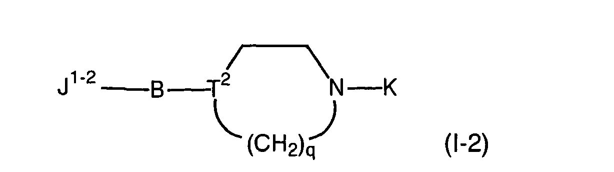
      where J¹⁻² is an indanonyl group which may have, as a substituent, a lower alkyl group with 1 to 6 carbon atoms or a lower alkoxy group with 1 to 6 carbon atoms; T² is a nitrogen atom; B, K and q are the same as defined above; (Japanese Patent No. 3078244, cl. 2)
   (vii) a cyclic amine derivative selected from the compounds represented by the following formulas or a pharmacologically acceptable salt thereof: 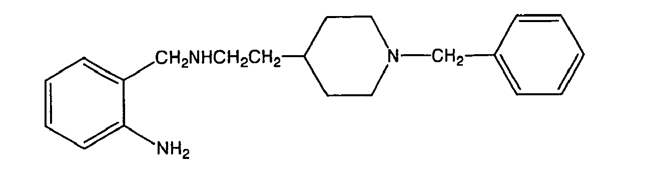 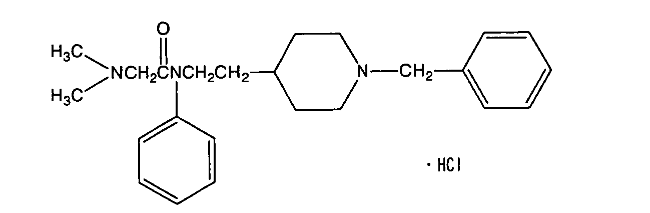 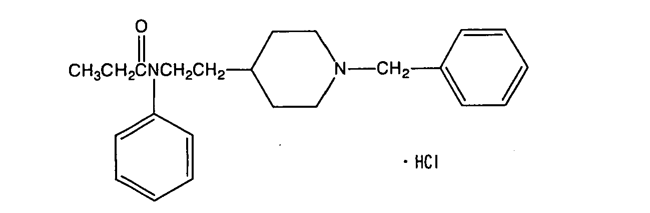 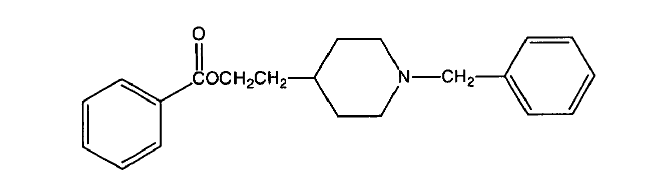 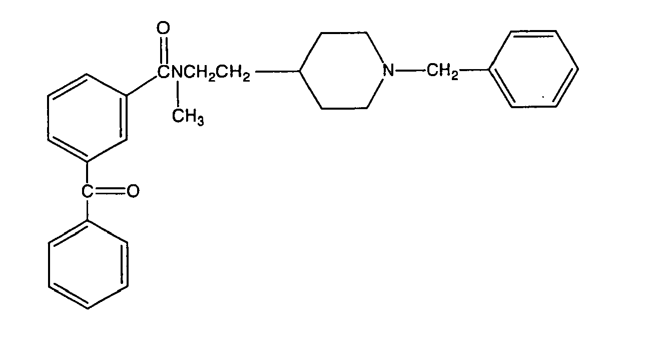 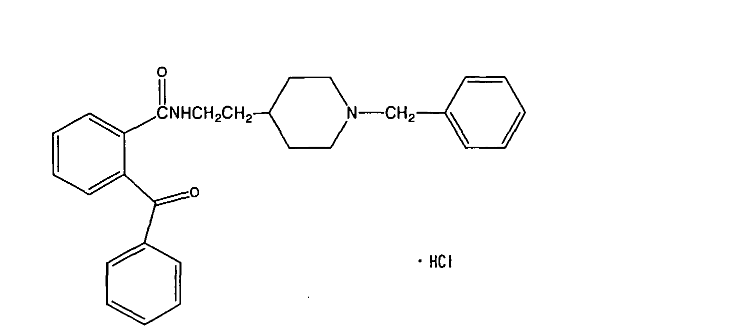 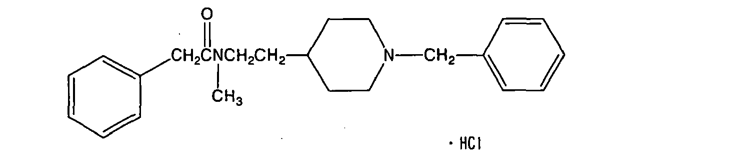 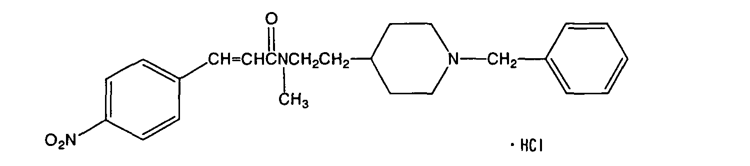 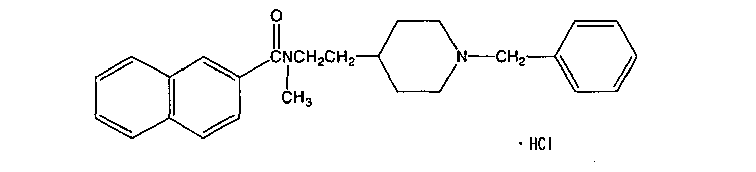 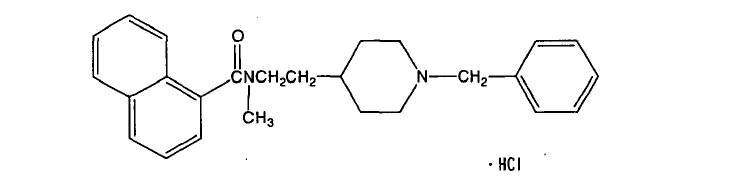 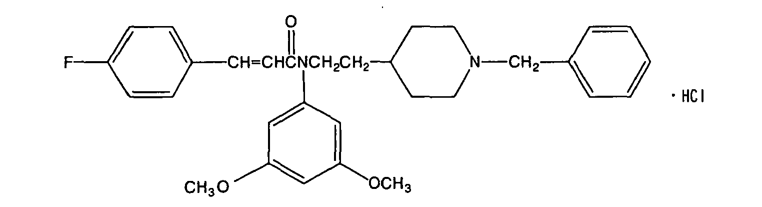 (Japanese Patent No. 3078244, cl. 4)
(2) A prophylactic and/or therapeutic agent for disorders in neurons of the central nervous system, comprising any one of the compounds shown in the following (i) to (vii):
   (i) 1-benzyl-4-[(5,6-dimethoxy-1-indanone)-2-yl]methylpiperidine represented by the following chemical formula or a pharmacologically acceptable salt thereof: 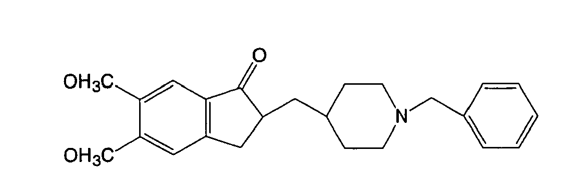 (Japanese Patent No. 2578475, cl. 1)
      The salts are preferably hydrochloride salts.
   (ii) a cyclic amine derivative represented by the following general formula (I) or a pharmacologically acceptable salt thereof: 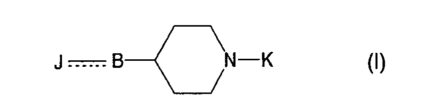 where J is a monovalent or divalent group selected from the groups represented by the following formulas: 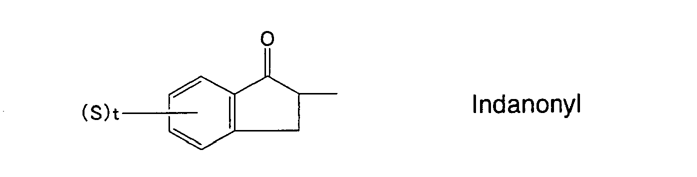 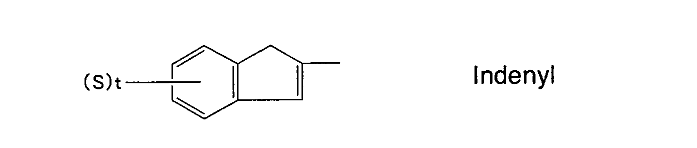 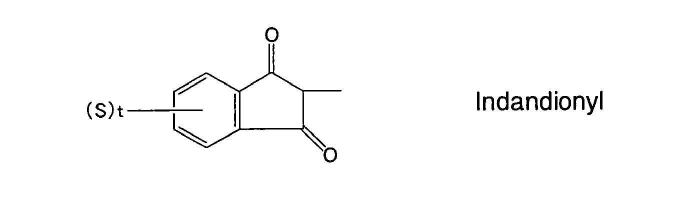 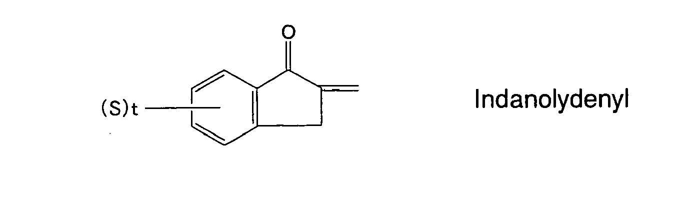 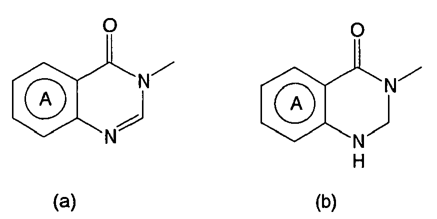 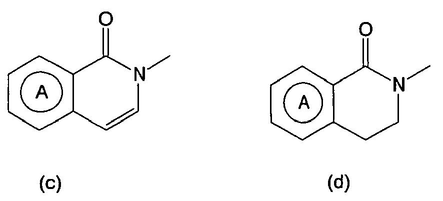 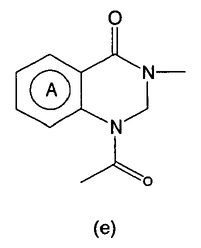 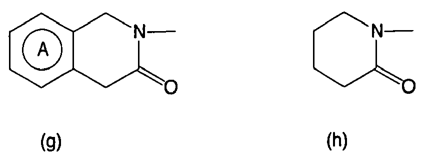 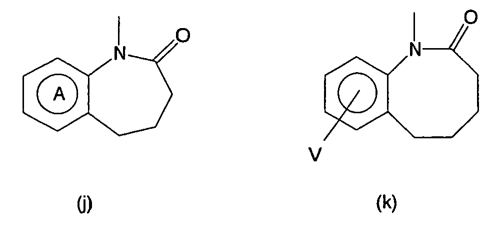 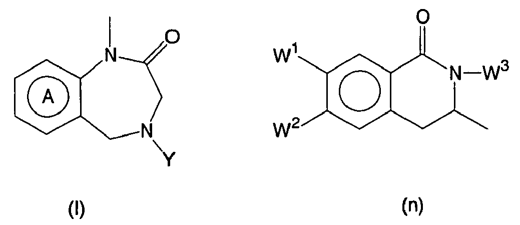 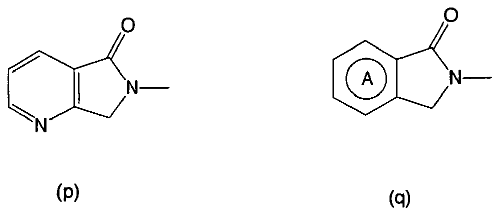 where S is a lower alkyl group with 1 to 6 carbon atoms, a lower alkoxy group with 1 to 6 carbon groups, a halogen atom or a hydroxyl group; t is 0 or an integer from 1 to 4; (S)ₜ may form a methylenedioxy or ethylenedioxy group between adjacent carbon atoms on the phenyl ring linked; Y in formula (1) is a hydrogen atom or a lower alkyl group with 1 to 6 carbon atoms; V in formula (k) is a hydrogen atom or a lower alkoxy group with 1 to 6 carbon atoms; W¹ and W² in formula (n) independently represent, similarly or differently, a hydrogen atom, a lower alkyl group with 1 to 6 carbon atoms, or a lower alkoxy group with 1 to 6 carbon atoms; W³ in formula (n) is a hydrogen atom or a lower alkyl group with 1 to 6 carbon atoms; phenyl ring A in formulas (a) to (e), (g), (j), (l) and (q) may be substituted with an alkyl group with 1 to 6 carbon atoms or a alkoxy group with 1 to 6 carbon atoms;
      B is a group represented by a formula -(CHR²)ₙ- (where n is 0 or an integer from 1 to 10; R² is each independently a hydrogen atom or a methyl group), a group represented by a formula =(CH-CH=CH)_{b}- (where b is an integer from 1 to 3), a group represented by a formula =CH-(CH₂)_{c}- (where c is 0 or an integer from 1 to 9), or a group represented by a formula =(CH-CH)_{d}= (where d is 0 or an integer from 1 to 5);
      K is a phenylalkyl group that may have, as a substituent, an alkyl group with 1 to 6 carbon atoms which may be halogenated, an alkoxy group with 1 to 6 carbon atoms, a nitro group, a halogen atom, a carboxyl group, a benzyloxy group, an alkoxycarbonyl group with 1 to 6 carbon atoms, an amino group, a monoalkylamino group with 1 to 6 carbon atoms, a dialkylamino group with 1 to 6 carbon atoms, a carbamoyl group, an acylamino group with 1 to 6 carbon atoms, a cyclohexyloxycarbonyl group, an alkylaminocarbonyl group with 1 to 6 carbon atoms, an alkylcarbonyloxy group with 1 to 6 carbon atoms, a hydroxyl group, a formyl group or an alkoxy (with 1 to 6 carbon atoms)-alkyl (with 1 to 6 carbon atoms) group; and 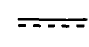 represents a single bond or a double bond;
      (Japanese Patent No. 2733203, cl. 1)
   (iii) a cyclic amine derivative selected from the compounds represented by the following formulas or a pharmacologically acceptable salt thereof: 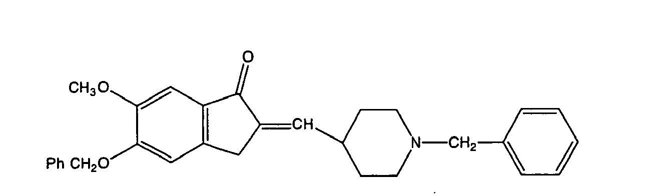 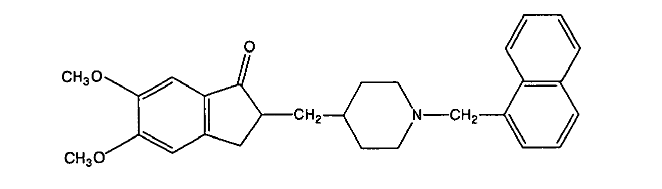 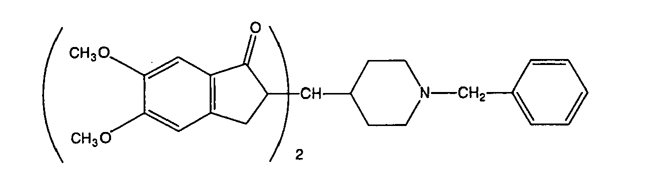 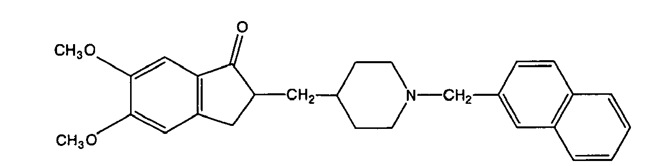 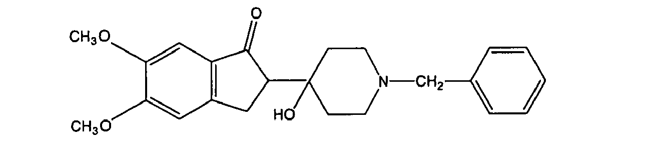 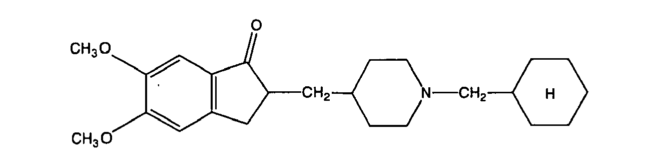 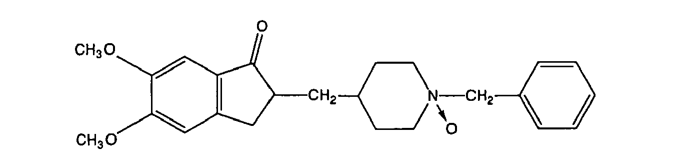 (Japanese Patent No. 2733203, cl. 7)
   (iv) a cyclic amine derivative selected from the compounds represented by the following formulas or a pharmacologically acceptable salt thereof: 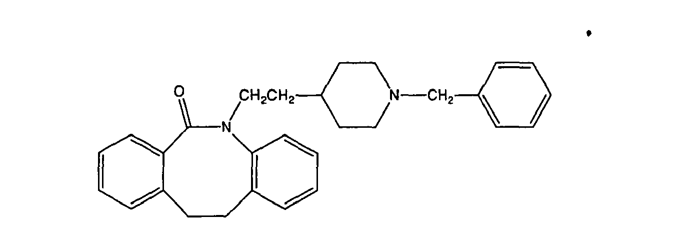 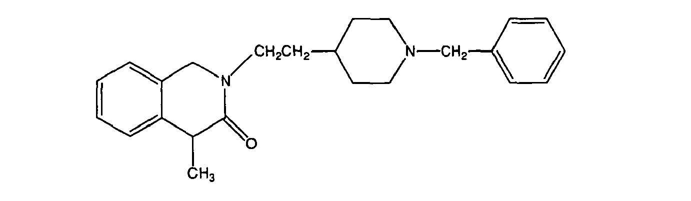 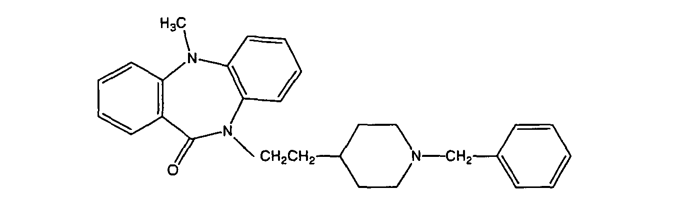 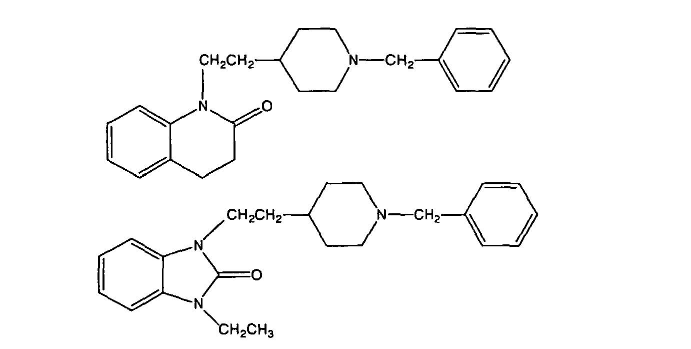 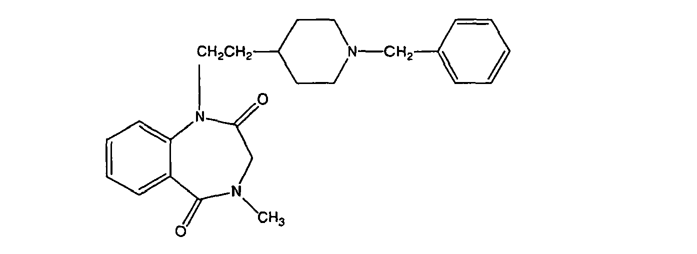 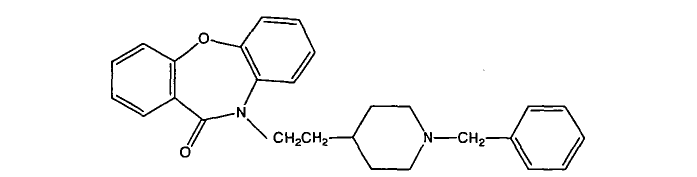 (Japanese Patent No. 2733203, cl. 8)
   (v) a cyclic amine derivative represented by the following general formula (I-1) or a pharmacologically acceptable salt thereof: 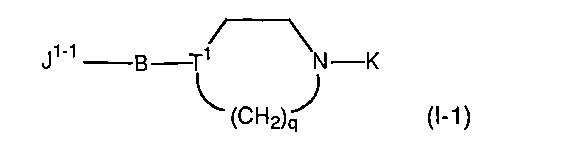 where J¹⁻¹ is a lower alkyl group with 1 to 6 carbon atoms (hereinafter, just referred to as "lower alkyl group"); a cyclohexyl group; a phenyl, pyridyl or pyrazyl group which may have, as a substituent, a lower alkyl group, a lower alkoxy group with 1 to 6 carbon atoms (hereinafter, just referred to as "lower alkoxy group"), a nitro group, a halogen, a carboxyl group, a lower alkoxycarbonyl group, an amino group, a mono-lower alkylamino group, a di-lower alkylamino group, a carbamoyl group, an acylamino group derived from aliphatic saturated monocarboxylic acid with 1 to 6 carbon atoms, a cyclohexyloxycarbonyl group, a lower alkylaminocarbonyl group, a lower alkylcarbonyloxy group, a halogenated lower alkyl group, a hydroxyl group, a formyl group or a lower-alkoxy-lower-alkyl group; a group represented by a formula 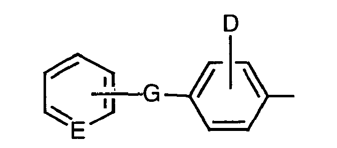 (where G is a group represented by a formula 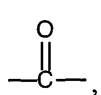 a group represented by a formula 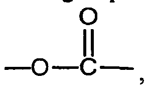 a group represented by a formula -O- , a group represented by a formula 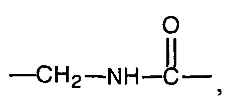 a group represented by a formula -CH₂-O-, a group represented by a formula -CH₂-SO₂- , a group represented by a formula 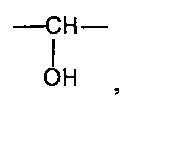 or a group represented by a formula 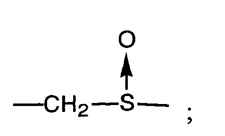 E is a carbon atom or a nitrogen atom);
      a quinolyl group; a quinoxalyl group; a furyl group or a group represented by a formula R¹-CH=CH- (where R¹ is a hydrogen atom or a lower alkoxycarbonyl group);
      B is a group represented by a formula -(CH₂)ₙ-, a group represented by a formula -NR²-(CH₂)ₙ- (where R² is a hydrogen atom, a lower alkyl group, a phenyl group or a lower alkylsulfonyl group), a group represented by a formula -CONR³-(CH₂)ₙ- (where R³ is a hydrogen atom, a lower alkyl group, a phenyl, benzyl or pyridyl group which may have, as a substituent, a lower alkyl group, a lower alkoxy group, a halogen or a hydroxyl group), a group represented by a formula -NH-CO-(CH₂)ₙ-, a group represented by a formula -CH₂-CO-NH-(CH₂)n-, a group represented by a formula -CO-CH₂-CH(OH)-CH₂-, a group represented by a formula -CO-(CH₂)ₙ-, a group represented by a formula -C(OH)-(CH₂)ₙ- or a group represented by a formula -CO-CH=CH-CH₂-; and n in the above formulas is 0 or an integer from 1 to 10;
      T¹ is a carbon atom;
      K is a phenylalkyl group (where the alkyl has 1 to 2 carbon atoms) in which the phenyl may have, as a substituent, a lower alkyl group, a lower alkoxy group, a nitro group, a halogen, a carboxyl group, a lower alkoxycarbonyl group, an amino group, a mono-lower alkylamino group, a di-lower alkylamino group, a carbamoyl group, an acylamino group derived from aliphatic saturated monocarboxylic acid with 1 to 6 carbon atoms, a cyclohexyloxycarbonyl group, a lower alkylaminocarbonyl group, a lower alkylcarbonyloxy group, a halogenated lower alkyl group, a hydroxyl group, a formyl group or a lower-alkoxy-lower-alkyl group; a cinnamyl group; a lower alkyl group; a pyridyl methyl group; a cycloalkyl (with 3 to 6 carbon atoms)-alkyl group; an adamantanemethyl group; a furfuryl group; a cycloalkyl group with 3 to 6 carbon atoms; or an acyl group; and
      q is 1 or 2; (Japanese Patent No. 3078244, cl.l)
   (vi) a cyclic amine derivative represented by the following general formula (1-2) or a pharmacologically acceptable salt thereof: 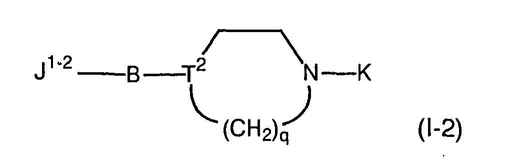
      where J¹⁻² is an indanonyl group which may have, as a substituent, a lower alkyl group with 1 to 6 carbon atoms or a lower alkoxy group with 1 to 6 carbon atoms; T² is a nitrogen atom; B, K and q are the same as defined above; (Japanese Patent No. 3078244, cl. 2)
   (vii) a cyclic amine derivative selected from the compounds represented by the following formulas or a pharmacologically acceptable salt thereof: 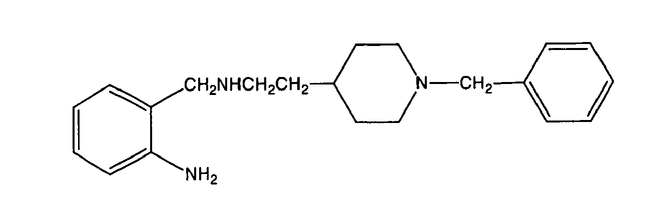 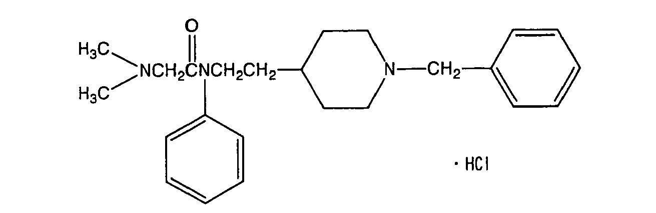 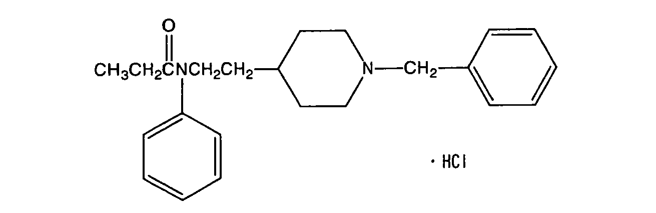 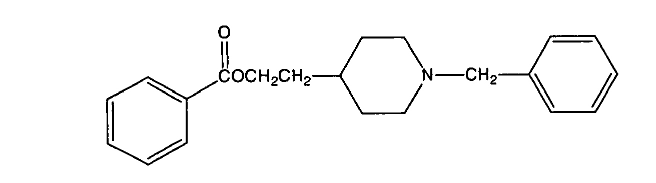 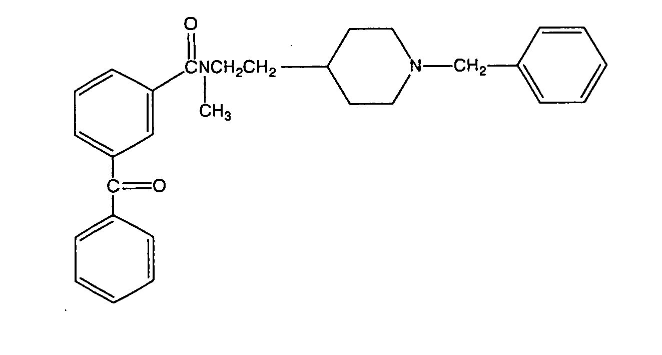 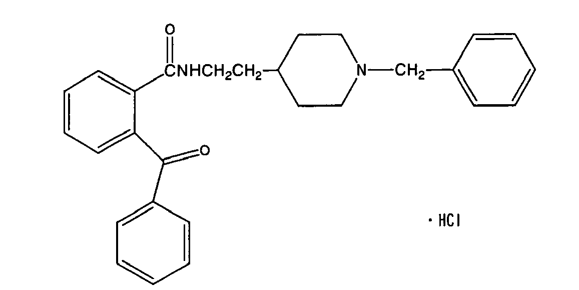 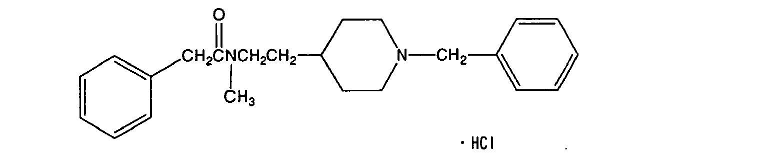 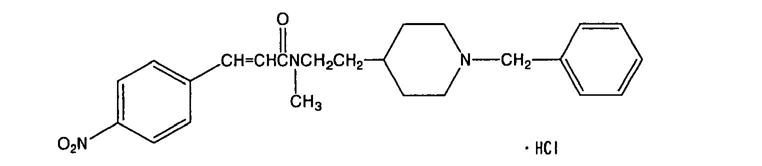 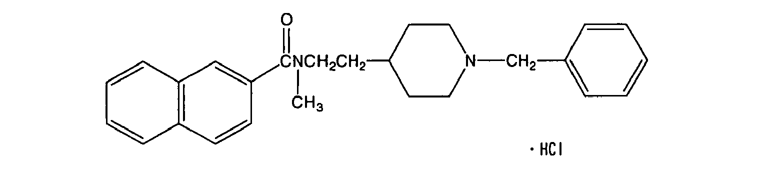 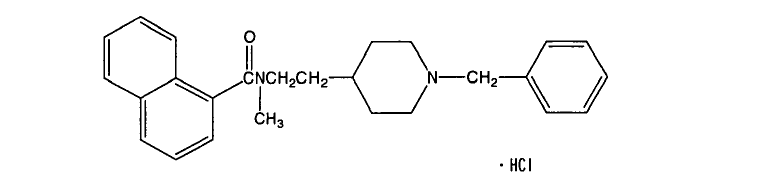 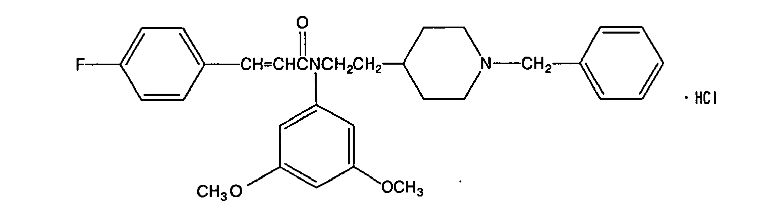 (Japanese Patent No. 3078244, cl. 4)
      In (1) and (2) described above, the salts of the compounds are preferably hydrochloride salts, in particular, donepezil hydrochloride represented by the following formula. 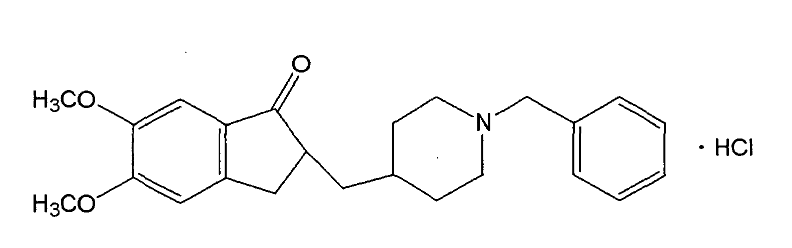
      In (2) above, the disorders in neurons include those disorders induced by cerebral ischemia, excitotoxicity or Aβ toxicity. As the cerebral ischemia or excitotoxicity, those associated with any one of cerebral apoplexy, cerebral infarction or cerebral embolism may be given. The Aβ toxicity may be associated with Alzheimer's disease or Down's syndrome.
      In (1) or (2) above, the cells are brain-derived, mature neurons; in particular, neurons derived from one selected from the group consisting of cerebral cortex, septal area and hippocampus may be given. These neurons may be primary culture cells.
(3) A prognosis improving agent for any disease selected from cerebral apoplexy, cerebral infarction or cerebral embolism, comprising the protective agent described in (1) above or the prophylactic and/or therapeutic agent described in (2) above.
(4) A method of protecting neurons of the central nervous system, comprising administering to a patient an effective amount of the protective agent described in (1) above.
(5) A method of preventing and/or treating disorders in neurons of the central nervous system, comprising administering to a patient an effective amount of the prophylactic and/or therapeutic agent described in (2) above.
   In (5) above, specific examples of disorders in neurons include those disorders induced by cerebral ischemia, excitotoxicity or Aβ toxicity. As the cerebral ischemia and excitotoxicity, those associated with any one of cerebral apoplexy, cerebral infarction or cerebral embolism may be given. Specific examples of excitotoxicity include one induced by NMDA or kainic acid. The Aβ toxicity may be associated with Alzheimer's disease or Down's syndrome.
(6) A method for improving the prognosis of any disease selected from cerebral apoplexy, cerebral infarction or cerebral embolism, comprising administering to a patient an effective amount of the prognosis improving agent described in (3) above.
(7) Use of any one of the compounds shown in (1) above for preparing any agent selected from the group consisting of the protective agent described in (1), the prophylactic and/or therapeutic agent described in (2), and the prognosis improving agent described in (3).
(8) A method of screening for a compound with Aβ aggregation inhibitory effect or a pharmacologically acceptable salt thereof, comprising contacting cholinergic neurons of the central nervous system with a candidate compound in the presence of Aβ and detecting or measuring the amount of Aβ aggregation.
   In the screening method of the present invention, it is possible to judge whether or not the candidate compound has Aβ aggregation inhibitory effect by comparing the results of detection or measurement of the amount of Aβ aggregation with the amount of Aβ aggregation in the absence of the candidate compound.
(9) A screening kit for a compound with Aβ aggregation inhibitory effect or a pharmacologically acceptable salt thereof, which is for use in the method described in (8) above.
(10) A method for screening for a compound, or a pharmacologically acceptable salt thereof, effective for preventing and/or treating disorders in neurons of the central nervous system induced by Aβ toxicity, comprising contacting cholinergic neurons of the central nervous system with a candidate compound in the presence of Aβ and detecting cytotoxicity or cell death.
   In the method of the present invention, it is possible to judge whether or not the candidate compound has cell protective effect against Aβ toxicity by comparing the results of detection of cytotoxicity or cell death with the extent of cytotoxicity or cell death in the absence of the candidate compound.
   The cytotoxicity or cell death mentioned above may be detected by measuring the concentration of lactate dehydrogenase (LDH) or by MTT assay.
(11) A screening kit for a compound, or a pharmacologically acceptable salt thereof, effective for preventing and/or treating disorders in neurons of the central nervous system induced by Aβ toxicity, which is for use in the method described in (10).

### BRIEF DESCRIPTION OF THE DRAWINGS

Fig. 1 is a graph showing LDH release from rat primary culture cells when donepezil was added 12 hours and 1 hour prior to oxygen glucose deprivation (OGD) treatment and 1 hour after OGD treatment. OGD(-) represents cells without OGD treatment, and OGD(+) represents cells with OGD treatment. "Cont" and "Vehi" are non-administration groups. The data were analyzed by Dunnett's test. Mark " *** " in Fig. 1 indicates P<0.005 ("vehicle group" is used as a control).
Fig. 2 shows the results of morphological observation of rat primary culture cells with or without OGD treatment. Panels A, B and C are photomicrographs showing the state of the following cells; A: control; B: cells with OGD treatment; C: cells with OGD treatment after addition of donepezil 12 hours prior to the treatment. Scale bar indicates 0.1 mm.
Fig. 3 is graphs showing LDH release from rat primary culture cells which received various acetylcholinesterase inhibitors or donepezil 12 hours prior to OGD treatment. OGD(-) represents cells without OGD treatment. OGD(+) represents cells with OGD treatment. "Cont" and "Vehi" are non-administration groups. The data were analyzed by Dunnett's test. Mark " * " in Fig. 3 indicates P<0.05; mark " ** " indicates P<0.01; and mark" *** " indicates P<0.005 ("vehicle group" is used as a control).
Fig. 4 is graphs showing LDH release from rat primary culture cells which received donepezil together with scopolamine (Sco) or mecamylamine (Mec) 12 hours prior to OGD treatment. OGD(-) represents cells without OGD treatment. OGD(+) represents cells with OGD treatment. "Cont" and "Vehi" are non-administration groups. The data were analyzed by ANOVA and Welch's t-test. Mark " * " in Fig. 4 indicates P<0.05; mark " ** " indicates P<0.01; and mark " *** " indicates P<0.005 ("vehicle group" is used as a control).
Fig. 5 is a graph showing the effect of donepezil on LDH release from rat primary culture cells induced by NMDA treatment. Donepezil was added 12 hours prior to the NMDA treatment. "Cont" and "Vehi" are non-administration groups. The data were analyzed by Dunnett's test. Mark " *** " in Fig. 5 indicates P<0.005 ("vehicle group" is used as a control).
Fig. 6 is a graph showing the effect of donepezil on LDH release from rat primary culture cells induced by kainic acid treatment. Donepezil was added 24 hours prior to the kainic acid treatment. "Cont" and "Vehi" are non-administration groups. The data were analyzed by Dunnett's test. Mark *** in Fig. 6 indicates P<0.005 ("vehicle group" is used as a control).
Fig. 7 shows immunostaining of cultured rat septal area neurons with anti-choline acetyltransferase antibody.
Fig. 8 is a graph showing the results of evaluation of the protective effect of donepezil on Aβ(1-40) toxicity using LDH as an indicator.
Fig. 9 shows immunostaining of cultured rat septal area neurons with anti-MAP2 antibody.
Fig. 10 is graphs showing the results of measurement of Aβ aggregation using CD changes as an indicator.
Fig. 11 is a graph showing the effect of siRNA on intracellular acetylcholine activity.
Fig. 12 is a graph showing the protective effect of siRNA on Aβ(1-40) toxicity.
Fig. 13 is graphs showing the aggregation inhibitory effect of siRNA on Aβ aggregation.

### BEST MODE FOR CARRYING OUT THE INVENTION

### 1. Outline

The present invention relates to a protective agent for neurons of the central nervous system and a prophylactic and/or therapeutic agent for disorders in neurons of the central nervous system, each comprising any one of the compounds shown below (hereinafter, referred to as the "compound Q"). According to the present invention, there are provided a method of protecting neurons of the central nervous system and/or a method of preventing and/or treating disorders in neurons of the central nervous system, each characterized by administering an appropriate amount of the prophylactic and/or therapeutic agent. Hereinbelow, the compound Q used in the protective agent, prophylactic agent, therapeutic agent and prognosis improving agent of the present invention will be described.

### 2. Compound Q

Cyclic amine derivative represented by the following general formula (I) and pharmacologically acceptable salts thereof (see Japanese Unexamined Patent Publication Nos. H1-79151, H07-252216 and H10-067739). 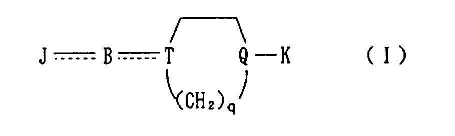 where J is:
(a) the following group which may be substituted or unsubstituted: (i) a phenyl group, (ii) a pyridyl group, (iii) a pyrazyl group, (iv) a quinolyl group, (v) a cyclohexyl group, (vi) a quinoxalyl group or (vii) a furyl group;
(b) a monovalent or divalent group selected from the following group in which the phenyl group may be substituted: (i) indanyl, (ii) indanonyl, (iii) indenyl, (iv) indenonyl, (v) indandionyl, (vi) tetralonyl, (vii) benzsuberonyl, (viii) indanolyl, (ix) a group represented by a formula 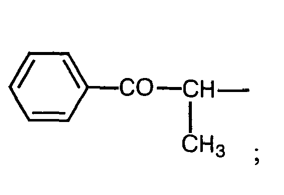
(c) a monovalent group derived from a cyclic amide compound;
(d) a lower alkyl group; or
(e) a group represented by a formula R¹-CH=CH- (where R¹ is a hydrogen atom or a lower alkoxycarbonyl group);
   B is a group represented by a formula 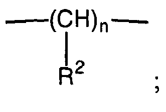 a group represented by a formula 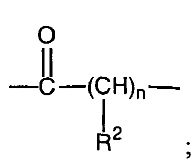 a group represented by a formula

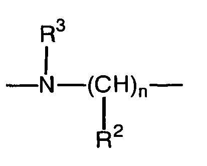

where R³ is a hydrogen atom, a lower alkyl group, an acyl group, a lower alkylsulfonyl group, a phenyl group which may be substituted, or a benzyl group;
a group represented by a formula 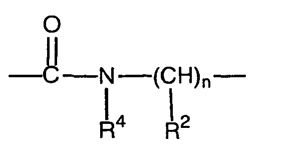
where R⁴ is a hydrogen atom, a lower alkyl group, or a phenyl group;
a group represented by a formula 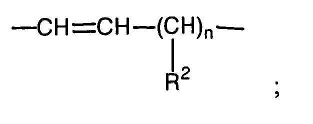 a group represented by a formula 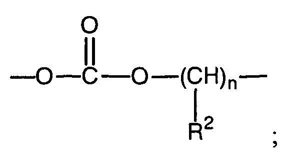 a group represented by a formula 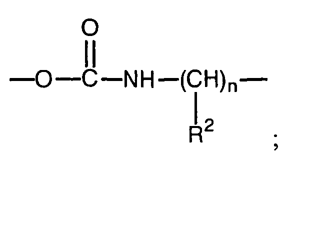 a group represented by a formula 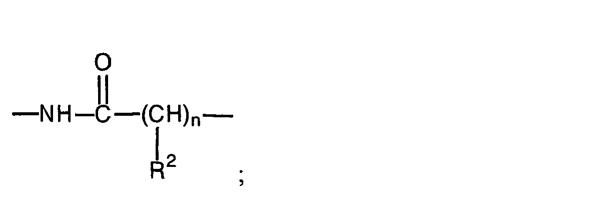 a group represented by a formula 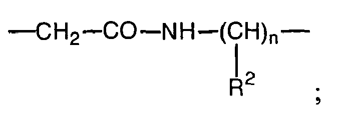 a group represented by a formula 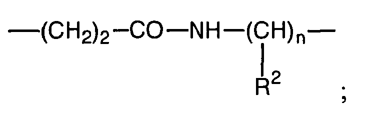 a group represented by a formula 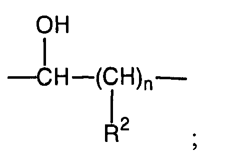 (in the above formulas, n is 0 or an integer from 1 to 10; and R² is a hydrogen atom or methyl group(s) in such a manner that the alkylene group represented by a formula 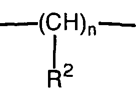 does not have a substituent or has one or more methyl groups);
a group represented by a formula =(CH-CH=CH)_{b}- (where b is an integer from 1 to 3);
a group represented by a formula =CH-(CH₂)_{c}- (where c is 0 or an integer from 1 to 9);
a group represented by a formula =(CH-CH)_{d}= (where d is 0 or an integer from 1 to 5);
a group represented by a formula 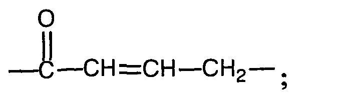 a group represented by a formula 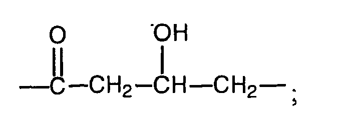 a group represented by a formula 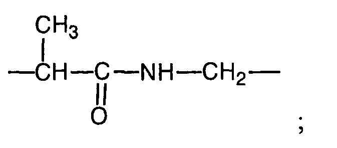 a group represented by a formula 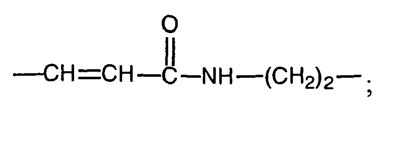 a group represented by a formula -NH-; a group represented by a formula -O-; a group represented by a formula -S-; a dialkylaminoalkylcarbonyl group or a lower alkoxycarbonyl group;
T is a nitrogen atom or a carbon atom;
Q is a nitrogen atom, a carbon atom or a group represented by a formula 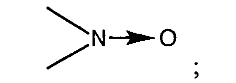
K is hydrogen atom, a substituted or unsubstituted phenyl group; an arylalkyl group in which the phenyl group may be substituted; a cinnamyl group in which the phenyl group may be substituted; a lower alkyl group; a pyridylmethyl group; a cycloalkylalkyl group; an adamantanemethyl group; a furylmethyl group; a cycloalkyl group; a lower alkoxycarbonyl group; or an acyl group;
q is an integer from 1 to 3;
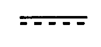 represents a single bond or a double bond.

In the above-mentioned definitions for compound (1), the lower alkyl group in J, K, R³ and R⁴ means a straight-chained or branched alkyl group with 1 to 6 carbon atoms, such as methyl group, ethyl group, propyl group, isopropyl group, butyl group, isobutyl group, sec-butyl group, tert-butyl group, pentyl group (amyl group), isopentyl group, neopentyl group, tert-pentyl group, 1-methylbutyl group, 2-methylbutyl group, 1,2-dimethylpropyl group, hexyl group, isohexyl group, 1-methylpentyl group, 2-methylpentyl group, 3-methylpentyl group, 1,1-dimethylbutyl group, 1,2-dimethylbutyl group, 2,2-dimethylbutyl group, 1,3-dimethylbutyl group, 2,3-dimethylbutyl group, 3,3-dimethylbutyl group, 1-ethylbutyl group, 2-ethylbutyl group, 1,1,2-trimethylpropyl group, 1,2,2-trimethylpropyl group, 1-ethyl-1-methylpropyl group, or 1-ethyl-2-methylpropyl group. Of these groups, preferable are methyl group, ethyl group, propyl group and isopropyl group. Methyl group is most preferable.

In the definition "the following group which may be substituted or unsubstituted: (i) a phenyl group, (ii) a pyridyl group, (iii) a pyrazyl group, (iv) a quinolyl group, (v) a cyclohexyl group, (vi) a quinoxalyl group or (vii) a furyl group" in J, specific examples of the substituent include lower alkyl groups with 1 to 6 carbon atoms, such as methyl group, ethyl group, n-propyl group, isopropyl group, n-butyl group, isobutyl group, or tert-butyl group; lower alkoxy groups corresponding to the above-mentioned lower alkyl groups, such as methoxy group or ethoxy group; nitro group; halogens such as chlorine, bromine or fluorine; carboxyl group; lower alkoxycarbonyl groups corresponding to the above-mentioned lower alkoxy groups, such as methoxycarbonyl group, ethoxycarbonyl group, isopropoxycarbonyl group, n-propoxycarbonyl group, or n-butyloxycarbonyl group; amino group; mono-lower alkylamino group; di-lower alkylamino group; carbamoyl group; acylamino groups derived from aliphatic saturated monocarboxylic acid with 1 to 6 carbon atoms, such as acetylamino group, propyonylamino group, butylylamino group, isobutylylamino group, valerylamino group or pivaloylamino group ; cycloalkyloxycarbonyl groups such as cyclohexyloxycarbonyl group; lower alkylaminocarbonyl groups such as methylaminocarbonyl group or ethylaminocarbonyl group; lower alkylcarbonyloxy groups corresponding to the above-defined lower alkyl groups, such as methylcarbonyloxy group, ethylcarbonyloxy group or n-propylcarbonyloxy group; halogenated lower alkyl groups represented by trifluoromethyl group; hydroxyl group; formyl group; and lower-alkoxy-lower-alkyl groups such as ethoxymethyl group, methoxymethyl group or methoxyethyl group. In the above explanation of the substituent, it is intended that the "lower alkyl group" and the "lower alkoxy group" include every group deriving from the above definitions. The group in J may be substituted with 1 to 3 substituents which may be the same or different.

Further, in the case of phenyl group, such a group as shown below is also included in the "substituted phenyl group" used herein:
A group represented by a formula 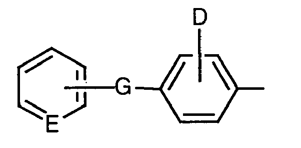 where G is a group represented by a formula 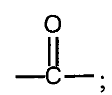 a group represented by a formula 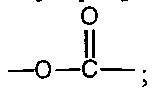 a group represented by a formula -O-; a group represented by a formula 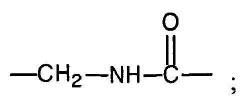 a group represented by a formula -CH₂-O-; a group represented by a formula -CH₂-SO₂-; a group represented by a formula 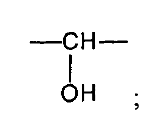 or a group represented by a formula 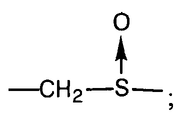 and
E is a carbon atom or a nitrogen atom.

Of those substituents, preferable substituents for phenyl group include, but are not limited to, lower alkyl groups, lower alkoxy groups, nitro group, halogenated lower alkyl groups, lower alkoxycarbonyl groups, formyl group, hydroxyl group, lower-alkoxy-lower-alkyl groups, halogen atoms, benzoyl group and benzylsulfonyl group. The substituents may be two or more, which may be the same or different.

Preferable substituents for pyridyl group include, but are not limited to, lower alkyl groups, amino group and halogen atoms.

Preferable substituents for pyrazyl group include, but are not limited to, lower alkoxycarbonyl groups, carboxyl group, acylamino group, carbamoyl group and cycloalkyloxycarbonyl group.

The pyridyl group as J is preferably 2-pyridyl group, 3-pyridyl group or 4-pyridyl group; the pyrazyl group as J is preferably 2-pyrazyl group; the quinolyl group as J is preferably 2-quinolyl group or 3-quinolyl group; the quinoxalyl group as J is preferably 2-quinoxalyl group or 3-quinoxalyl group; and the furyl group as J is preferably 2-furyl group.

In the definition of J, representative examples for groups (i) to (ix) described in (b) are as follows.

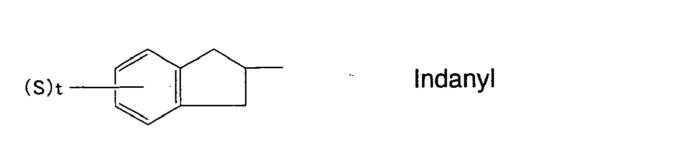

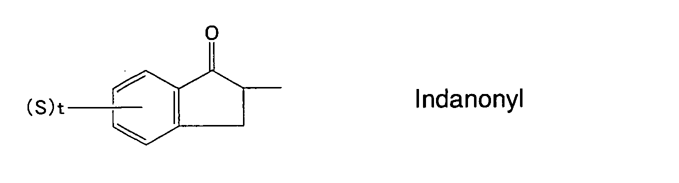

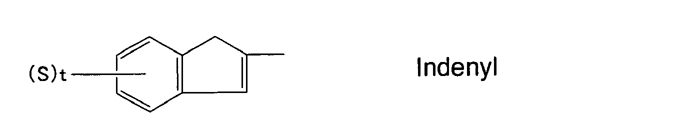

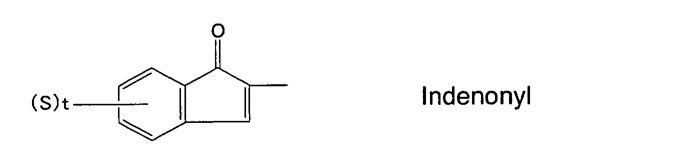

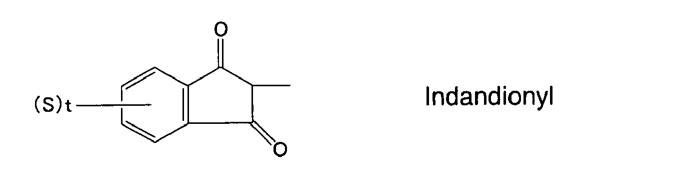

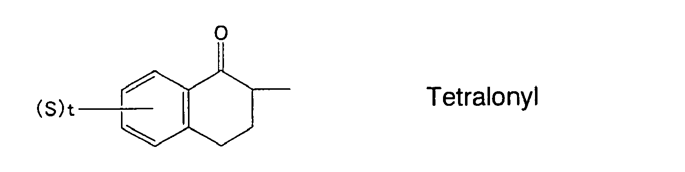

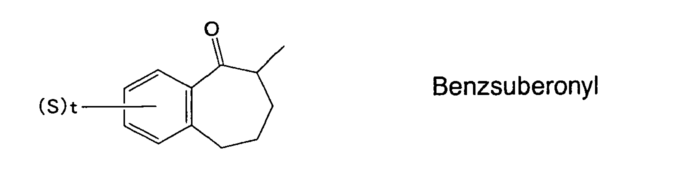

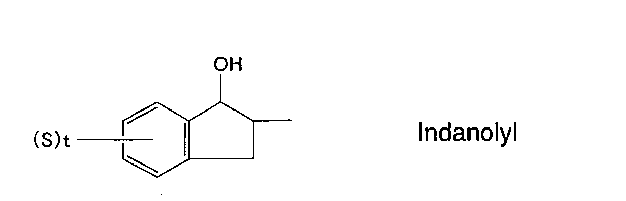

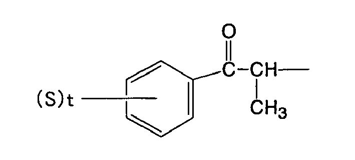

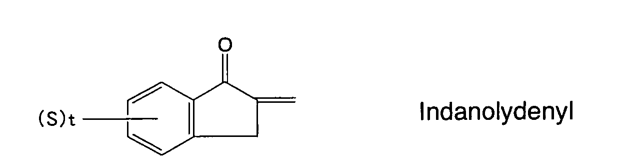

In the above-described series of formulas, t is 0 or an integer from 1 to 4; and S is one of the substituents (which may be the same or different) defined in (a) in J or a hydrogen atom. Preferably, S is a hydrogen atom (unsubstituted), a lower alkyl group or a lower alkoxy group. Further, S may be substituted with an alkylenedioxy group (such as methylenedioxy group or ethylenedioxy group) between adjacent carbon atoms on the phenyl ring.

Most preferably, S is not substituted or have 1 to 3 methoxy groups substituted.

The indanolydenyl shown above is one example of the divalent group in which the phenyl group may be substituted in the definition of (b) in J. Indanolydenyl is a representative divalent group derived from indanonyl of (ii) in (b) in J.

In the definition of J, the monovalent group derived from circular amide compounds includes, but is not limited to, quinazolone, tetrrahydroisoquinoline-one, tetrahydrobenzodiazepine-one and hexahydrobenzazocine-one. Any monovalent group having a circular amide in its structural formula may be included.

The circular amide can be derived from a single ring or a condensed hetero-ring. Preferably, the condensed hetero-ring is one condensed with a phenyl ring. In this case, the phenyl ring may be substituted with lower alkyl groups with 1 to 6 carbon atoms (preferably methyl group), lower alkoxy groups with 1 to 6 carbon atoms (preferably methoxy group) or halogen atoms.

Preferable examples are given below.

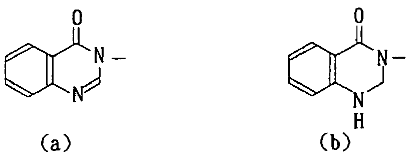

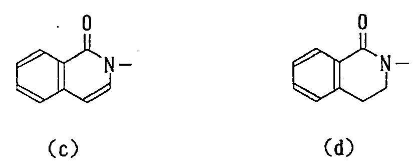

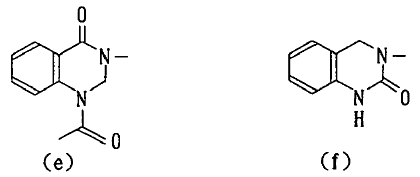

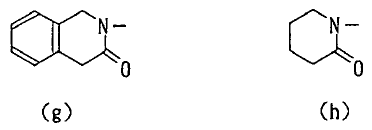

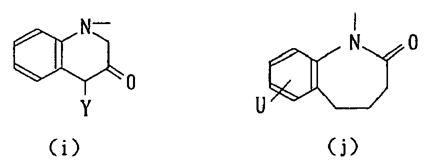

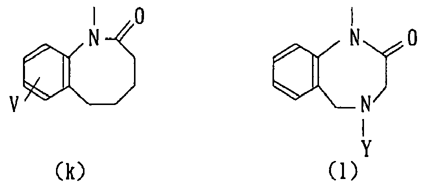

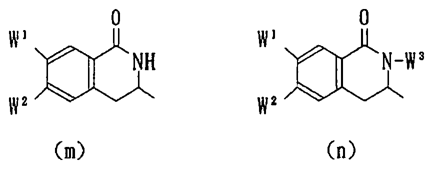

In the above formulas, Y in formulas (i) and (1) is a hydrogen atom or a lower alkyl group; V in formula (k) is a hydrogen atom or a lower alkoxy group; W¹ and W² in formulas (m) and (n) represent a hydrogen atom, a lower alkyl group or a lower alkoxy group; and W³ in formula (n) represents a hydrogen atom or a lower alkyl group.

In formulas (j) and (l), the ring on the right is a seven-membered ring. In formula (k), the ring on the right is a eight-membered ring.

Among the definitions of J described above, the most preferable is a monovalent group derived from indanone in which the phenyl ring may be substituted or a monovalent group derived from a circular amide compound.

In the definition of B, the group represented by a formula 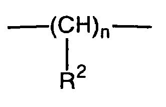 is represented by a formula -(CH₂)ₙ- when R² is a hydrogen atom; further, this formula means that one or more methyl groups may be linked to any of the carbon atoms of the alkylene chain. In this case, n is preferably. 1 to 3.

In the series of formulas shown in the definition of B, a group having an amide group is also a preferable group.

Examples of more preferable groups include a group represented by a formula =(CH-CH=CH)_{b}- (where b is an integer from 1 to 3); a group represented by a formula =CH-(CH₂)_{c}- (where c is 0 or an integer from 1 to 9); a group represented by a formula =(CH-CH)_{d}= (where d is 0 or an integer from 1 to 5); a group represented by a formula -NH-; a group represented by a formula -O-; or a group represented by a formula -S-. Ring 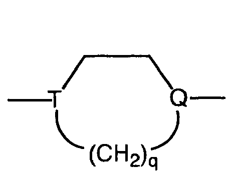 may be a five- to seven-membered ring. Specifically, 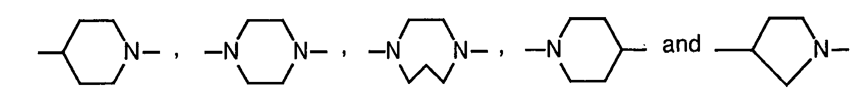 may be given. A particularly preferable ring is a piperidine ring represented by a formula

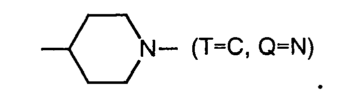

In the terms "substituted or unsubstituted phenyl group" and "substituted or unsubstituted arylalkyl group" used in the definition of K, the substituent is the same as defined in (i) to (vii) in (a) in the definition of J.

The arylalkyl group means benzyl group, phenethyl group, etc. in which the phenyl group is substituted with the above-mentioned substituent or not substituted.

Specific examples of pyridylmethyl group include 2- pyridylmethyl group, 3-pyridylmethyl group and 4- pyridylmethyl group.

Most preferable K is an arylalkyl group in which the phenyl group may be substituted, a substituted or unsubstituted phenyl group, or a cinnamyl group in which the phenyl group may be substituted.

Specific examples of preferable arylalkyl group include benzyl group and phenethyl group in which the phenyl group may be substituted with a lower alkoxy group with 1 to 6 carbon atoms, a lower alkyl group with 1 to 6 carbon atoms, a hydroxyl group, etc. 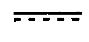 represents a single bond or a double bond.

As an example where the bond is a double bond, the above-described divalent group derived from indanone in which the phenyl ring may be substituted, i.e., indanolydenyl group may be given.

In the present invention, specific examples of pharmacologically acceptable salts include inorganic acid salts such as hydrochloride salts, sulfate salts, hydrobromate salts and phosphorate salts; and organic acid salts such as formate salts, acetate salts, trifluoroacetate salts, maleate salts, tartarate salts, methanesulfonate salts, benzenesulfonate salts and toluenesulfonate salts.

Depending on the selection of substituents, the compound may form alkali metal salts such as sodium salts and potassium salts; alkaline earth metal salts such as calcium salts and magnesium salts; organic amine salts such as trimethylamine salts, triethylamine salts, pyridine salts, picoline salts, dicyclohexylamine salts and N,N'-dibenzylethylenediamine salts; and ammonium salts.

In the present invention, the above-described compound may have asymmetric carbon atoms depending on the type of the substituent. Thus, optical isomers may exist. Needless to say, such optical isomers are included in the scope of the present invention.

In one specific example when J has the indanone skeleton, J has asymmetric carbon atoms and thus geometrical isomers, optical isomers, diastereomers, etc. may exist. Such geometrical isomers, optical isomers, diastereomers, etc. are included in the scope of the present invention.

Taking all the definitions into consideration, a group of compounds especially preferable are as described below. A cyclic amine represented by the formula (A) or a pharmacologically acceptable salt thereof: 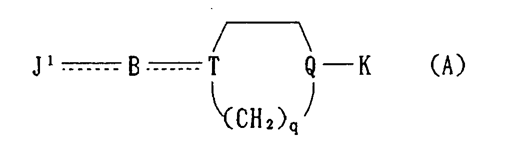 where J¹ is a monovalent or divalent group selected from the following group in which the phenyl group may be substituted: (i) indanyl, (ii) indanonyl, (iii) indenyl, (iv) indenonyl, (v) indandionyl, (vi) tetralonyl, (vii) benzsuberonyl, (viii) indanolyl or (ix) a group represented by a formula 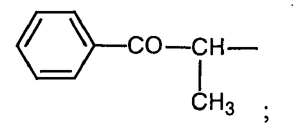 B, T, Q, q and K are the same as defined above.

In the definition of J¹ described above, most preferable group is an indanonyl, indandionyl or indanolydenyl group in which the phenyl group may be substituted. In this case, most preferably, the phenyl group is unsubstituted or substituted with the same or different substituents that are hydroxyl groups, halogen atoms and lower alkoxy groups. Examples of lower alkoxy groups include methoxy group, ethoxy group, isopropoxy group, n-propoxy group and n-butoxy group each having 1 to 6 carbon atoms. They may be mono-to tetra-substituted. Preferably, the lower alkoxy group is di-substituted. Most preferable is di-substituted methoxy group.

A group of compounds which are included in formula (A) above and are still more preferable can be represented by the following general formula (B). 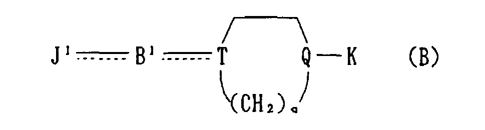 where J¹ is a monovalent or divalent group selected from the following group in which the phenyl group may be substituted: (i) indanyl, (ii) indanonyl, (iii) indenyl, (iv) indenonyl, (v) indandionyl, (vi) tetralonyl, (vii) benzsuberonyl, (viii) indanolyl or (ix) a group represented by a formula 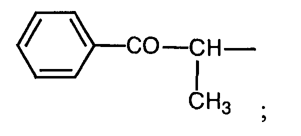 B¹ is a group represented by a formula 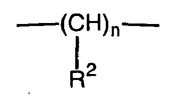 where n is 0 or an integer from 1 to 10; R² is a hydrogen atom or a methyl group in such a manner that the alkylene group represented by a formula 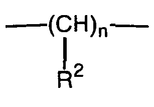 does not have a substituent or has one or more methyl groups;
a group represented by a formula =(CH-CH=CH)_{b}- (where b is an integer from 1 to 3);
a group represented by a formula =CH-(CH₂)_{c}- (where c is 0 or an integer from 1 to 9);
a group represented by a formula =(CH-CH)_{d}= (where d is 0 or an integer from 1 to 5);
T, Q, q and K are the same as defined above.

A group of compounds which are included in formula (B) above and are still more preferable can be represented by the following general formula (C). 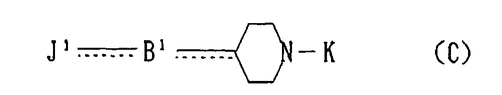 where J¹, B¹ and K are the same as defined above.

This formula corresponds to a case where the group represented by the formula 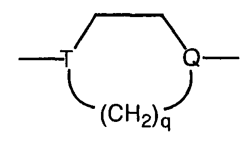 is the group represented by a formula 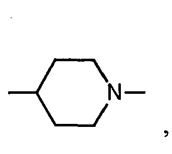 i.e., piperidine.

A group of compounds which are included in formula (C) above and are still more preferable can be represented by the following general formula (D). 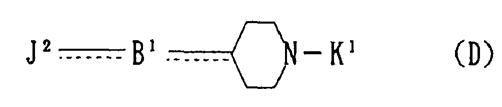 where J² is a group selected from an indanonyl, indandionyl or indanolydenyl group in which the phenyl group may be substituted;
K¹ is a substituted or unsubstituted phenyl group, an arylalkyl group which may be substituted, or a cinnamyl group which may be substituted; and
B¹ is the same as defined above.

### 3. Protection of Neurons

In the present invention, "neurons of the central nervous system" means brain-derived, mature neurons (preferably, human- or other mammal-derived neurons of the central nervous system which have been involved in neuronal network and matured functionally); they are derived from one selected from the group consisting of cerebral cortex, septal area and hippocampus.

In the present invention, protection of neurons of the central nervous system means protective effect upon neurons of the central nervous system against various loads resulted from ischemia-like effect such as cerebral ischemia; resulted from excitotoxicity induced by excitant substances such as NMDA (N-methyl-D-aspartate) or kainic acid; resulted from peptide or protein aggregate toxicity (including Aβ toxicity and prion toxicity; resulted from NO (nitric oxide) or active oxygen species; and so forth. Disorders of neurons induced by cerebral ischemia include disorders induced by degenerative diseases, etc. of cerebral neurons such as cerebral apoplexy, cerebral infarction or cerebral embolism (which is said recently to be the cause of Alzheimer's disease). The above-mentioned peptide or protein aggregate toxicity (especially Aβ toxicity) includes those which are induced in association with Alzheimer's disease or Down's syndrome.

The above-mentioned "protection of neurons" is used in a wide meaning. For example, protection of neurons include not only to actually prevent the death of neurons of the central nervous system caused by loads on them (e.g., physical or chemical disorders undesirable for the maintenance of homeostasis, such as stress, trophopathy, diseases, injuries, decreased strength due to surgical operations or the like, prostration, aging; or cytotoxicity) but also to prevent the lowering of functions of neurons.

In order to examine whether neurons have been protected or not, neurons must be prepared. The neurons for this purpose are not particularly limited. They may be primary culture cells prepared from biological samples. With respect to preparation of primary culture cells from biological samples, for example, cerebral cortex-derived primary culture neurons may be prepared from cerebral cortex, and septal area-derived primary culture neurons may be prepared from the septal region, i.e., the region including septal area and basal forebrain.

Animal species from which cells are collected are not particularly limited. For example, rat, mouse, guinea pig, hamster, rabbit or the like may be used. Although cells may be taken at any stage of growth from embryo to adult, it is preferable to take cells from embryos (e.g., 18-day-old embryos). Tissues taken are treated with such as trypsin or collagenase to thereby obtain neurons.

Cell culture may be performed by conventional methods for culturing animal cells. One of ordinary skill in the art can select appropriate culture conditions.

### 4. Protective Agent for Neurons and Method of Protection

Compound Q described earlier has protective effect against disorders of neurons (especially, neurons of the central nervous system). Also, the siRNA of acetylcholinesterase gene (AChE gene) inhibits the expression of AChE gene to thereby inhibit the function of AChE, and thus inhibits the aggregation of Aβ. Therefore, in the present invention, compound Q or the siRNA of AChE gene described later is useful as an active ingredient in a protective agent for neurons of the central nervous system against degenerative diseases of cerebral neurons such as cerebral apoplexy, cerebral infarction or cerebral embolism, and a prophylactic, therapeutic and/or prognosis improving agent for disorders of neurons (hereinafter, sometimes referred to as the "protective agent, etc. of the present invention"). A therapeutic agent, treating method, prognosis improving agent and prognosis improving method for Alzheimer's disease and Down's syndrome are provided by the present invention.

The active ingredient of the protective agent etc. of the present invention (e.g., donepezil hydrochloride) may be either an anhydride or a hydrate. Further, the above donepezil can have crystal polymorphism, for example. In this case, a single crystal form or a mixture of crystal forms may be used.

Compound Q used in the present invention (e.g., donepezil hydrochloride) may be prepared by known methods. For example, the compound can be prepared easily by the method disclosed in Japanese Unexamined Patent Publication No. H1-79151, Japanese Patent No. 2578475, Japanese Patent No. 2733203 or Japanese Patent No. 3078244. Donepezil hydrochloride is also available as a preparation in the form of fine granules or the like.

The siRNA used in the present invention is designed by the method described later. One of ordinary skill in the art can prepare the siRNA by known methods using a nucleic acid synthesizer or the like.

It is possible to use compound Q or the siRNA of AChE gene as it is in the protective agent etc. of the present invention. Alternatively, it is also possible to formulate the compound or the siRNA into a preparation together with known, pharmacologically acceptable carriers and the like. Examples of pharmacologically acceptable carriers include excipients, binders, disintegrants, lubricants, coloring agents, flavoring agents, stabilizers, emulsifiers, absorption enhancer, surfactants, pH adjusters, preservatives, antioxidants, etc. Forms of preparations include tablets, powders, fine granules, granules, capsules, syrups and the like for oral administration and suppositories, injections, ointments, poultices and the like for parenteral administration.

The route of administration of the protective agent etc. of the present invention is not particularly limited. The protective agent may be administered orally or parenterally. For example, oral dosage forms of donepezil hydrochloride are available as Aricept™ fine granules (Eisai) and Aricept™ tablets (Eisai). With respect to forms of parenteral administration, transdermal absorption, intravenous injection, subcutaneous injection, intradermal injection, intramuscular injection, intraperitoneal injection and the like may be enumerated. Intravenous injection is preferable. Injections may be prepared as non-aqueous diluents (e.g., glycols such as propylene glycol, polyethylene glycol, vegetable oils such as olive oil, alcohols such as ethanol), suspensions or emulsions. The asepticizing of injections may be performed by filter sterilization, addition of sterilants, or the like. Alternatively, injections may take a form which is prepared at the time of use. That is, an aseptic solid composition is prepared by lyophilization or the like, and this composition is dissolved in solvent such as aseptic distilled water for injections before use. When the protective agent etc. of the present invention is administered by transdermal absorption in the form of a patch, it is preferable to select the so-called free form which does not form salts.

The dose of compound Q in oral administration is preferably 0.1-100 mg/day, more preferably 1.0-50 mg/day, taking donepezil hydrochloride as an example. Preferable dos of the siRNA of AChE gene is 0.01-100 mg/day, more preferably 0.1-50 mg/day.

In parenteral administration, when patches are used, the dose is preferably 5-50 mg/day, more preferably 10-20 mg/day. In the case of injections, they may be prepared by dissolving or suspending in a pharmacologically acceptable carrier (such as physiological saline or commercial distilled water for injections) to give a concentration of 0.1 µg/ml carrier to 10 mg/ml carrier. The dose of the thus prepared injection is 0.01-5.0 mg/day, more preferably 0.1-1.0 mg/day for patients who need treatment. This dose may be administered at once or divided into two or three administrations per day.

### 5. Method of Evaluating Performance

The effect of the protective agent of the invention on neurons against ischemic disorders can be evaluated by OGD (oxygen glucose deprivation) test. Further, the protective agent of the invention on neurons against excitotoxicity can be evaluated by NMDA or kainic acid stimulation test. Further, the protective agent of the invention on neurons against Aβ toxicity can be evaluated by Aβ toxicity test or Aβ aggregation test. Hereinbelow, methods of individual tests will be described.

### (1) OGD Test

In OGD test, a model is used in which ischemia-like cytotoxicity is induced in rat primary culture cerebral cortex neurons by giving a load via oxygen glucose deprivation. Whether donepezil hydrochloride has protective effect on neurons against the ischemia-like disorder or not is examined using that model.

In this Example, primary culture neurons can be prepared from the cerebral cortex of rat embryos (17-19-day-old embryos). Cells which have been cultured for more than 7 days under conventional culture conditions for animal cells (e.g., at 37°C under 5% CO₂ may be used. OGD treatment is performed by rat primary culture cerebral cortex neurons in a glucose-free buffer and transferring them into a tightly sealed chamber where nitrogen replacement is carried out to create a low oxygen environment. Cells after OGD treatment are transferred from the glucose-free buffer to a cell culture medium and cultured overnight at 37°C under 5% CO₂.
(A) In order to clarify whether compound Q (e.g., donepezil) has protective effect on neurons against ischemic disorders or not, first, this Example is performed under conditions where the compound is added before and after the OGD treatment. For example, neurocyte protective effects are compared among the following groups: "Pre-12h" where donepezil was added 12 hours before OGD treatment; "Pre-1h" where donepezil was added 1 hour before OGD treatment; "Post-1h" where donepezil was added 1 hour after OGD treatment; "Cont" where no OGD treatment was given; and "Vehicle" where no donepezil was added before or after OGD treatment. As an indicator for the effect of neurocyte protection, ratio of LDH release inhibition can be used. LDH (lactate dehydrogenase) is an oxidation-reduction enzyme present in cytoplasm and converts pyruvic acid into lactic acid to thereby reduce the amount of intracellular NADH (nicotinamide adenine dinucleotide). Therefore, when cells are injured by OGD, LDH flows out from inside of the cells into extracellular solution. In this solution, LDH is present depending on the degree of injury of the cells (cell death). The amount of LDH present in the solution can be determined by adding pyruvic acid and NADH to the solution and measuring the decreasing ratio of NADH with an absorption spectrometer. According to this testing example, it is revealed that the highest neurocyte protection effect is produced when donepezil was added 12 hours before OGD treatment ("Pre-12h").
(B) It is also possible to observe the degree of injury to the cells or the degree of protection of neurons by the compound by microscopically observing the morphology of the OGD treated, rat primary culture neurons. When compared with control cells, vehicle-treated cells do not take the normal form. On the other hand, when donepezil was added before OGD treatment, serious injury as seen in vehicle-treated cells is not observed and cells show a form close to that seen before OGD treatment. Therefore, from the viewpoint of cellular morphology, it is also demonstrated that neurons are protected by adding donepezil before OGD treatment.
(C) Further, another test is possible. Briefly, rat primary culture neurons are subjected to OGD treatment in the same manner as in the above-described test. At that time, various acetylcholinesterase inhibitors (galantamine, tacrine and rivastigmine) and donepezil are added to the cells in varied concentrations to examine their effect upon LDH release. In this test, while the acetylcholinesterase 50% inhibition concentration of donepezil is almost equivalent to that of rivastigmine, rivastigmine and other acetylcholinesterase inhibitors did not show any neurocyte protective effect. This suggests that the protective effect of donepezil on OGD treated neurons is based on a mode of action which is different from acetylcholinesterase inhibitory action.
(D) Subsequently, in order to examine whether the above-mentioned protective effect of donepezil on neurons is mediated by acetylcholine receptor or not, acetylcholine receptor antagonists scopolamine (muscarine receptor antagonist) and mecamylamine (nicotine receptor antagonist) are added to cells to see how the protective effect of donepezil will be affected. According to this test, the protective effect of donepezil on neurons is not affected by the addition of scopolamine and mecamylamine; thus, it is demonstrated that the effect of donepezil is not mediated by acetylcholine receptor.

### (2) Excitotoxicity Test

### (A) NMDA Toxicity

In excitotoxicity test, protective effects of compounds (e.g., donepezil) on primary culture neurons against NMDA (N-methyl-D-aspartate) toxicity are examined. A model is used in which cytotoxicity is induced in rat primary culture cerebral cortex neurons by NMDA stimulation. Briefly, NMDA is added (e.g., 100 µM for 9 hours) to primary culture neurons obtained as described above and then the amount of LDH in the culture medium is measured. Donepezil is added to cells before the addition of NMDA stimulation, e.g., 12 hours before the NMDA stimulation. Then, after addition of NMDA, the amount of LDH in the culture medium may be measured, for example, 9 hours after the addition and used as an indicator for neurocyte protection effect.

According to this test, it is clear that donepezil shows protective effect on neurons against NMDA excitotoxicity in a concentration-dependent manner.

### (B) Kainic Acid Toxicity

It is said that kainic acid enhances the death of neurons induced by β-amyloid which is said one of the causative factors of Alzheimer's disease. In this test, protective effects of compounds (e.g., donepezil) on rat primary culture cerebral cortex neurons against cytotoxicity induced by kainic acid are examined.

Kainic acid treatment may be performed, for example, by adding kainic acid to the medium of cells which have been cultured for 7 days or more and then culturing the cells overnight at 37°C under 5% CO₂. Compound Q (e.g., donepezil) may be added to the cells, for example, 24 hours before the addition of kainic acid stimulation. After addition of kainic acid, the amount of LDH present in the culture medium is measured, for example, 24 hours after the addition and used as an indicator for neurocyte protection effect. In this test, donepezil inhibits the LDH release of neurons in a dose-dependent manner. Therefore, it is clear that donepezil shows protective effect against kainic acid excitotoxicity in a concentration-dependent manner.

### (3) Aβ Toxicity Test or Aβ Aggregation Test

### (A) Cultured Septal Area Neurons

Aβ is considered to be a cause of Alzheimer's disease, and central cholinergic nerves involved in memory and learning are lost in Alzheimer's disease. And cholinergic nerves are projected from septal area to regions such as hippocampus that are susceptible to disorders in Alzheimer's disease. Taking into consideration the above-described findings and the fact that volume reduction in hippocampus caused by Alzheimer's disease is inhibited by administration of donepezil hydrochloride, it is preferable to analyze the protective effect against Aβ toxicity and Aβ aggregation inhibitory effect of compounds (e.g., donepezil) in cholinergic nerves.

A great number of septal area neurons have choline acetyltransferase (ChAT) (an acetylcholine synthesis enzyme) and thus are believed to be cholinergic nerves. The present invention has demonstrated for the first time in the above-mentioned septal area neurons (which are cholinergic nerves) that the Aβ aggregation inhibitory effect of donepezil protects cholinergic neurons against toxicity.

Septal area neurons may be prepared by taking cells from living bodies and culturing them. They may be derived from any one of mouse, rat, guinea pig, hamster, rabbit, or the like; the species is not particularly limited. Although cells may be taken at any stage of growth from embryo to adult, it is preferable to take cells from embryos (e.g., 18-day-old embryos). The septal region of the brain (i.e., the region including septal area and basal forebrain) is cut out and treated with trypsin to thereby obtain cells.

Septal area neurons may be obtained by plating the thus obtained cells on culture plates at an appropriate concentration and culturing them. Preferably, the culture plates are coated with poly-D-lysine. The cell concentration is preferably 1.2 x 10⁵ cells per well of, for example, 96-well poly-D-lysine-coated plates. As the medium, 5% fetal calf serum-containing DMEM may be used. Still preferably, DMEM contains 5 µg/ml insulin, 30 nmol/L sodium selenite, 100 µmol/L putrescine, 20 nmol/L progesterone, 15 nmol/L biotin, 100 units/ml penicillin, 100 µg/ml streptomycin, 1 mmol/L sodium pyruvate, and the like.

The confirmation that cultured septal area neurons are cholinergic nerves can be made using the expression of ChAT as an indicator as described above. The presence or absence of ChAT expression can be confirmed by immnunostaining the cultured septal area neurons with anti-ChAT antibody.

### (B) Detection of Aβ Aggregation

In the present invention, Aβ aggregation can be examined using as an indicator changes in CD (circular dichroism) spectra at 215-260 nm attributable to the formation of a β-sheet structure of Aβ. CD spectra at 215-260 nm decrease when Aβ forms an α-helix structure or a β-sheet structure. In particular, it is known that the formation of a β-sheet structure decreases CD spectra around 215 nm.

In another embodiment, fluorescence of Aβ may be measured with thioflavin T to examine Aβ aggregation in culture medium simply. Briefly, 48 hours after addition of Aβ(1-42) to cells, medium samples are taken and 10 µmol/L of thioflavin T is added thereto. Immediately thereafter, fluorescence is measured with an excitation wavelength of 450 nm and an emission wavelength of 490 nm (Wall J., Schell M., Murphy C., Hrncic R., Stevens F.J., Solomon A. (1999) Thermodynamic instability of human lambda 6 light chains: correlation with fibrillogenicity, *Biochemistry* 38(42), 14101-14108).

### (C) Detection of Aβ Toxicity

It is known that when Aβ takes a β-sheet structure, Aβ readily forms mass of Aβ fiber and shows toxicity. In the present invention, detection of Aβ toxicity may be performed using known methods in which cytotoxicity is measured after addition of Aβ to septal area neurons. Preferable, representative examples of such methods include, but are not limited to, those described below. For example, Aβ toxicity is examined using the amount of LDH in the medium as an indicator in the same manner as in (1) above. Alternatively, in the present invention, Aβ toxicity is examined by MTT [(dimethylthiazol-2-yl)-2,5-diphenyltetrazolium bromide] assay after addition of Aβ. MTT assay may be performed, for example, by adding MTT (Sigma) to the medium to give a final concentration of 1 mg/ml, incubating the medium at 37°C for 1 hour, removing the medium, solubilizing cells with DMSO, and measuring the absorbance at 550 nm. Alternatively, Aβ toxicity is examined by a cytotoxicity measuring method using alamar blue. In this method, alamar blue (Wako Purechemical) is added to the medium at 10% and reacted under 5% CO₂. Four hours after the addition, absorbance was measured with an excitation wavelength of 530 nm, a fluorescence wavelength of 590 nm and gain 35. Measured results are converted taking the result for medium alone as 0% and the result for control as 100%.

The Aβ added to cells may be the full-length Aβ or Aβ(1-40) consisting of the N-terminal 40 residues. As long as the sequence takes a β-sheet structure, Aβ of any length may be used.

Alternatively, the degree of cell death caused by Aβ toxicity may be examined by immunostaining with anti-MAP2 antibody. Since MAP2 is a neurocyte marker, it is possible to confirm the degree of loss of neurons induced by Aβ toxicity from the amount of cells expressing MAP2. The degree of cell death may also be examined by a method of measuring cytotoxicity using trypan blue. Briefly, trypan blue is added to cells, and those cells which are not stained with dark blue are counted as livable cells.

### (D) Nerve Protective Agent against Aβ Toxicity and Aβ Aggregation Inhibitor for Septal Area Neurons

When the siRNA of acetylcholinesterase (AChE) gene is added to septal area neurons, intracellular AChE activity decreases. By measuring the LDH described in (C) above, it is demonstrated that disorders of septal area neurons induced by Aβ toxicity is ameliorated by treatment with siRNA.

Further, by measuring the CD spectra described in (B), it is demonstrated that Aβ aggregation is inhibited by the treatment with siRNA in the siRNA-added septal area neurons.

From what has been described above, it is clear that AChE is involved in the Aβ toxicity and Aβ aggregation in septal area neurons. Therefore, an Aβ Aggregation inhibitor comprising a compound which has an effect of inhibiting the function of acetylcholinesterase and also has an effect of substantially inhibiting the interaction between acetylcholinesterase and Aβ is included in the present invention.

The term "interaction" means the binding of acetylcholinesterase to Aβ. The expression "substantially inhibiting the interaction between acetylcholinesterase and Aβ" means to inhibit the enhancement of Aβ aggregation in the presence of acetylcholinesterase. And as long as Aβ toxicity is inhibited, the interaction can be said "substantially" inhibited.

The lowering of the function of acetylcholine may be brought by inhibition of the activity of acetylcholinesterase or by inhibition of the expression of acetylcholinesterase gene.

For the inhibition of the activity of AChE, any compound which inhibits the enzyme activity of AChE may be used as long as it inhibits the interaction between acetylcholine and Aβ (e.g., donepezil hydrochloride represented by the following formula). Alternatively, anti-AChE antibody may be used.

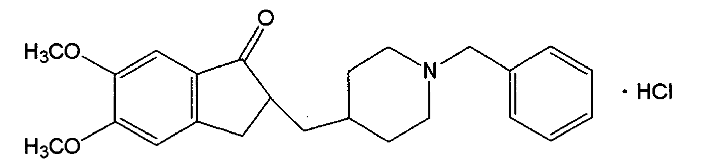

For the inhibition of the expression of AChE gene, any nucleic acid inducing RNAi may be used (e.g., siRNA or shRNA) as long as the nucleic acid inhibits the interaction between acetylcholine and Aβ and yet inhibits the expression of AChE gene. In order to inhibit the expression of AChE gene by RNAi, for example, an siRNA (small interfering RNA) or shRNA (short hairpin RNA) for AChE gene may be designed and synthesized. Then, the siRNA or shRNA may be allowed to act to induce RNAi. siRNA is a short-strand RNA produced intracellularly through the processing of Dicer. On the other hand, shRNA is a small RNA molecule folded into a hairpin structure and has a stem-loop structure in which the sense strand and the antisense strand are linked with a loop.

Standards for siRNA design are as follows.
(a) A region downstream of the initiation codon of a gene encoding AChE is selected. Any sequence located downstream of the initiation codon may be used; any region may be the candidate.
(b) From the selected region, a sequence of consecutive 11-30 bases, preferably 21-25 bases, starting with aa is selected. Specifically, sequences which comprise one of the following nucleotide sequences as a target sequence and consist of 30 bases or less (e.g., 11-30 bases, preferably 21-25 bases) may be used as siRNA (combination of (i-1) and (i-2); and combination of (ii-1) and (ii-2)).

(i-1) 5'-AAUGUCAGUGACUCUGUUUTT-3' (SEQ ID NO: 1)

(i-2) 5'-AAACAGAGUCACUGACAUUTT-3'    (SEQ ID NO: 2)

(ii-1) 5'-GCUGCCUCAAGAAAGUAUCTT-3'    (SEQ ID NO: 3)

(ii-2) 5'-GAUACUUUCUUGAGGCAGCTT-3' (SEQ ID NO: 4)

In the present invention, the siRNA of AChE gene is preferably used in the form of a double-stranded RNA formed by the combination of (i-1) and (i-2) or the combination of (ii-1) and (ii-2).

Standards for shRNA design are as follows.
(a) shRNA sequences for AChE to be used for RNAi are designed in a region specific to the gene, i.e., from the 5'-terminal region to 300 bases (Elbashir, S.M. et. al. Genes Dev. 15, 188-200 (2001)).
(b) From a region near 5' end of individual gene transcripts, mRNA sequences starting with aa or a are selected and designed as target sequences. The length of the target region is not particularly limited. The length is preferably 30 bp or less, more preferably 21-25 bp.

In order to introduce siRNA or shRNA into cultured septal area neurons, various methods may be used. For example, a method in which *in vitro* synthesized siRNA or shRNA is linked to a plasmid DNA and this plasmid is introduced into the neurons; or a method of annealing two strands of RNA may be used.

Further, compounds which are predictable from the structure analysis of Aβ aggregation inhibitory effect-showing siRNA or shRNA and have Aβ aggregation inhibitory effect are also included in the present invention.

### (E) Detection of Aβ Aggregation Inhibitory Effect and Protective Effect against Aβ Toxicity by Cholinesterase Inhibitor or siRNA of Cholinesterase Gene

The present invention provides a method of screening for compounds with Aβ aggregation inhibitory effect or pharmacologically acceptable salts thereof. Briefly, it is possible to screen for those compounds which inhibit Aβ aggregation by adding a test compound (candidate compound) to neurons of the central nervous system in the presence of Aβ and then measuring decrease in CD spectra at 215-260 nm, especially at 215 nm. The judgment whether the test compound has Aβ aggregation inhibitory effect or not can be made by comparing the amount of Aβ aggregation in the presence of the test compound with the amount of Aβ aggregation in the absence of the test compound. The present invention also provides a screening kit for compounds with Aβ aggregation inhibitory effect or pharmacologically acceptable salts thereof, to be used for the above-described method.

The kit of the present invention contains what are necessary for measuring Aβ aggregation inhibition by the CD spectrum method described in (B) above. With respect to Aβ necessary for Aβ aggregation and reagents used in the measurement of CD spectra, those used in Examples 6 and 7 described later may be used appropriately.

Further, the present invention provides a method of screening for compounds, or pharmacologically acceptable salts thereof, effective in preventing and/or treating disorders in neurons of the central nervous system induced by Aβ toxicity. Briefly, it is possible to screen for compounds effective in preventing and/or treating disorders in neurons of the central nervous system induced by Aβ toxicity by adding Aβ and a test compound to cultured septal area neurons and then detecting cytotoxicity or cell death. The cytotoxicity or cell death can be detected by measuring the amount of LDH described in (C) above or by MTT assay or the like. The present invention also provide a screening kit for compounds, or pharmacologically acceptable salts thereof, effective in preventing and/or treating disorders in neurons of the central nervous system induced by Aβ toxicity, to be used in the above-described method.

The kit of the present invention contains what are necessary for measuring disorders in neurons induced by Aβ toxicity using the amount of extracellular LDH described in (C) above as an indicator, or what are necessary for performing MTT assay or the like. With respect to reagents necessary for determining the degree of cytotoxicity induced by Aβ toxicity with LDH, those used in the following Examples 1, 2, 3, 5 and 7 may be used appropriately. For example, NADH and pyruvic acid may be used. With respect to reagents necessary for determining the degree of cytotoxicity induced by Aβ toxicity with MTT, MTT may be used.

These components constituting the kit may be provided separately. Alternatively, they may be provided together as long as there is no hindrance. The kit may contain solutions, or components to prepare such solutions may be in a condensed form.

Further, if necessary, the kit may also comprise adjuvants, exclusive containers, other necessary accessories, manuals, and the like.

In the present invention, the Aβ aggregation inhibitory effect of acetylcholinesterase inhibitor (such as donepezil) or the siRNA of acetylcholinesterase gene can be confirmed by adding the acetylcholinesterase inhibitor before (e.g., 24 hours) adding Aβ and measuring the CD spectra described above after (e.g., 48 hours) the addition of Aβ.

Further, the protective effect against Aβ toxicity of acetylcholinesterase inhibitor (such as donepezil) or the siRNA of acetylcholinesterase gene can be confirmed by measuring the amount of LDH in the medium of cells treated in the same manner as described above.

Hereinbelow, the present invention will be described more specifically with reference to the following Examples which should not be construed as limiting the present invention.

### EXAMPLE 1: OGD Test

In this Example, a model is used in which ischemia-like cytotoxicity is induced in rat primary culture cerebral cortex neurons by giving a load via oxygen glucose deprivation. Whether donepezil hydrochloride has protective effect on neurons against the ischemia-like disorder or not is examined using that model.

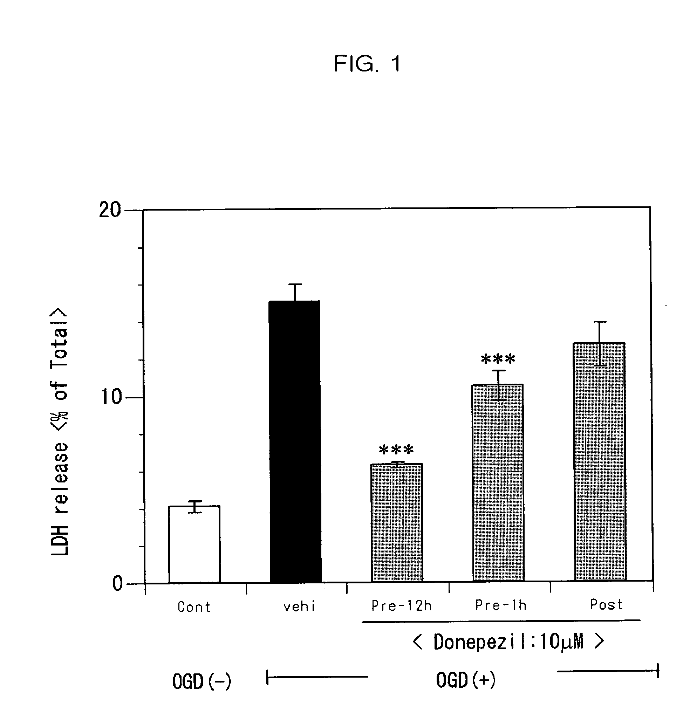

In this Example, primary culture neurons were prepared from the cerebral cortex of rat embryos (17-19-day-old embryos). As a culture medium, DMEM (Gibco BRL) supplemented with 10% fetal calf serum (Gibco BRL), 10% horse serum (Gibco BRL), 5 µg/ml insulin (Sigma), 30 nM sodium selenite (Sigma), 100 µM putrecine (Sigma), 20 nM progesterone (Sigma), 15 nM biotin (Sigma), 100 units/ml penicillin (Gibco BRL), 100 µg/ml streptomycin (Gibco BRL), 8 mM glucose and 1 mM pyruvic acid (Sigma) was used (Scholtz et al., 1988). Cells were cultured at 37°C under 5% CO₂. After culturing for 7 days or more, cells were subjected to OGD treatment. Briefly, rat primary culture cerebral cortex neurons were placed in a glucose-free buffer (Krebs-Ringer bicarbonate buffer: 5.36 mM KCl, 1.26 mM CaCl₂, 0.44 mM KH₂PO₄, 0.49 mM MgCl₂, 0.41 mM MgSO₄, 137 mM NaCl, 4.17 mM NaHCO₃, 0.34 mM Na₂HPO₄, 10 mM HEPES (pH 7.4)) and then transferred into a tightly sealed chamber where nitrogen replacement was carried out to create a low oxygen environment. Cells after OGD treatment are transferred from the glucose-free buffer to a cell culture medium and cultured overnight at 37°C under 5% CO₂. First, donepezil was added before and after OGD treatment to examine whether neurocyte protective effect is observed or not, or whether neurons change or not. Neurocyte protective effects were compared among the following groups: "Pre-12h" where donepezil was added 12 hours before OGD treatment; "Pre-1h" where donepezil was added 1 hour before OGD treatment; "Post-1h" where donepezil was added 1 hour after OGD treatment; "Cont" where no OGD treatment was given; and "Vehicle" where no donepezil was added before or after OGD treatment. As an indicator for neurocyte protection effect, ratio of LDH release inhibition was used. LDH (lactate dehydrogenase) is an oxidation-reduction enzyme present in cytoplasm and converts pyruvic acid into lactic acid to thereby reduce the amount of intracellular NADH (nicotinamide adenine dinucleotide). Therefore, when cells are injured by OGD, LDH flows out from inside of the cells into extracellular solution. In this solution, LDH is present depending on the degree of injury of the cells (cell death). The amount of LDH present in the solution can be determined by adding pyruvic acid and NADH to the solution and measuring the decreasing ratio of NADH with an absorption spectrometer. The results are shown in Fig. 1. As clearly seen from Fig. 1, "Pre-12h" showed the highest LDH release inhibitory effect, and "Pre-1h" and "Post-1h" followed in this order. From these results, it is understood that donepezil protects neurons effectively when added 12 hours before OGD treatment. The time point of donepezil addition was fixed at Pre-12h in any of the subsequent OGD tests.

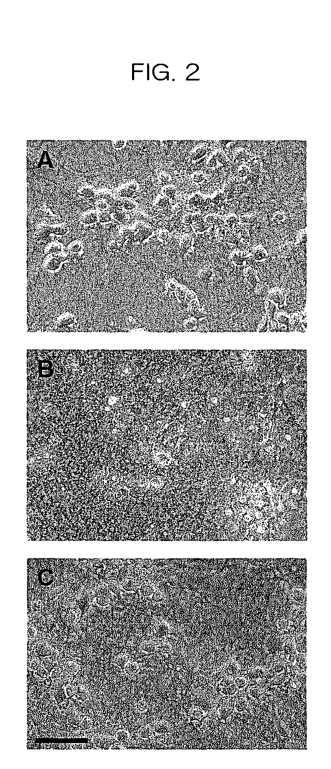

Subsequently, rat primary culture neurons which had been subjected to OGD treatment in the same manner as in the previous test were microscopically observed. The results are shown in Fig. 2. As clearly seen from Fig. 2, cells in Vehicle (B) do not show the normal state, as compared with control (A). It is remarkable that cells in Vehicle have been damaged by OGD. On the other hand, when donepezil was added before OGD treatment, such damage as seen in (B) was not observed and cells are clearly in a state close to that of neurons before OGD treatment (C). Thus, it was demonstrated that neurons were protected by the addition of donepezil before OGD treatment.

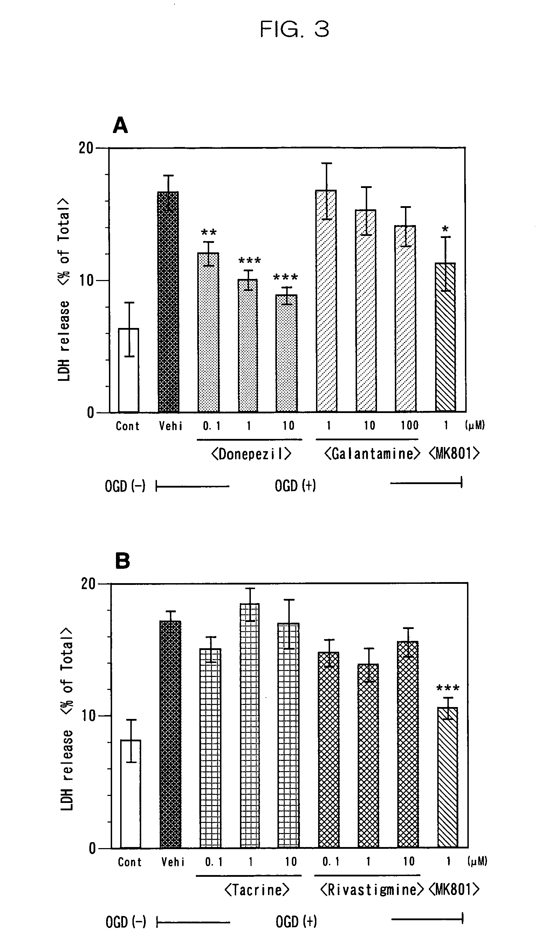

Subsequently, rat primary culture neurons were subjected to OGD treatment in the same manner as in the previous test. At that time, various acetylcholinesterase inhibitors (galantamine, tacrine and rivastigmine) and donepezil were added to the cells in varied concentrations to examine their effect upon LDH release. The concentrations used were 0.1, 1.0 and 10 µM for donepezil, tacrine and rivastigmine; and 1.0, 10 and 100 µM for galantamine. The results are shown in Fig. 3. As clearly seen from Fig. 3A, only donepezil inhibits statistically significantly the LDH release of neurons in a dose-dependent manner. The 50% inhibition concentrations (IC₅₀) of acetylcholinesterase inhibitors in disrupted suspension of rat brain are shown in Table 1.

**Table 1**

| AChE Inhibition | IC₅₀ (nM) |
|---|---|
| Donepezil | 6.7±0.35 |
| Galantamine | 1200±33 |
| Tacrine | 77 ±1.4 |
| Rivastigmine | 4.3±0.087 |

| | |
|---|---|
| Ogura et al. 2000. Comparison of inhibitory activities of Donepezil and other cholinesterase inhibitors on acetylcholinesterase and butyrylcholinesterase in vitro. | |
| Methods Find Exp Clin Pharmacol. 22, 609-613 | |

As is clear from Table 1, the acetylcholinesterase 50% inhibition concentration of donepezil is almost equivalent to that of rivastigmine. However, rivastigmine and other acetylcholinesterase inhibitors did not show any neurocyte protective effect. These results suggest that the neurocyte protective effect of donepezil is based on a mode of action which is different from acetylcholinesterase inhibitory action.

Subsequently, how the neurocyte protective effect of donepezil is affected when acetylcholine receptor antagonists scopolamine (muscarine receptor antagonist) and mecamylamine (nicotine receptor antagonist) are added to cells was examined by the following experiment.

Briefly, in the same manner as in the previous tests, donepezil, scopolamine, mecamylamine, a combination of donepezil and scopolamine, or a combination of donepezil and mecamylamine was added to the culture medium of neurons 12 hours before OGD treatment. Then, their effects on LDH release induced by OGD treatment were tested. Each of donepezil, scopolamine and mecamylamine was added at the concentration of 10 µM.

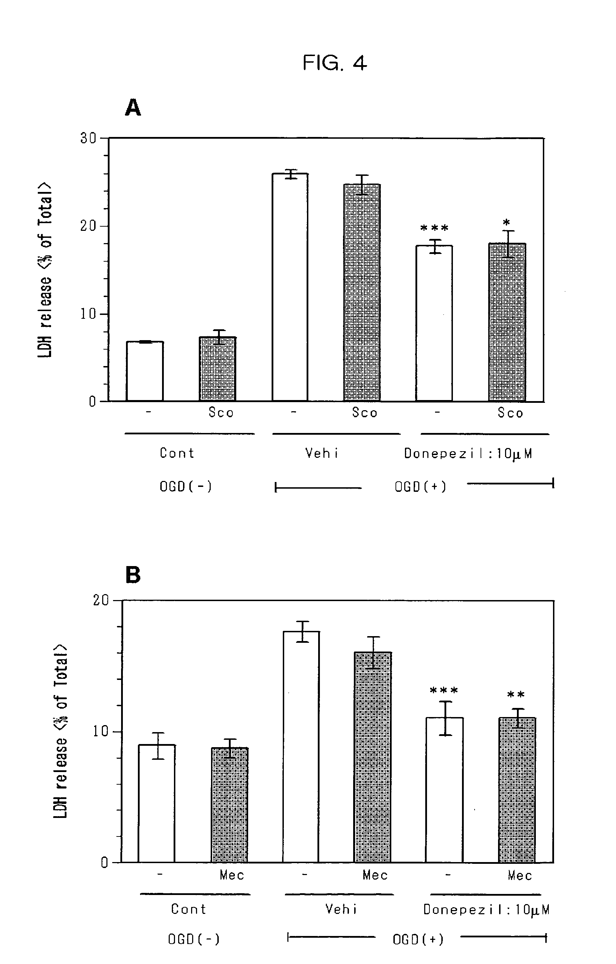

The results are shown in Fig. 4. From Fig. 4, it is clear that the neurocyte protective effect of donepezil is not affected by the addition of scopolamine (Fig. 4A) and mecamylamine (Fig. 4B) in any of the samples of "Cont" (without OGD treatment), "Vehi" (without donepezil) and donepezil-added samples. This indicates that donepezil is protecting neurons regardless of the presence or absence of inhibition by acetylcholine receptors. Therefore, according to this experiment, it is believed that donepezil acts neurocyte protectively by a mode of action other than acetylcholinesterase inhibitory action.

### EXAMPLE 2: Excitotoxicity Test

In this Example, the neurocyte protective effect of donepezil against NMDA toxicity was examined. A model is used in which cytotoxicity is induced in rat primary culture cerebral cortex neurons by NMDA stimulation. Briefly, 100 µM of NMDA was added to rat primary culture neurons obtained in the same manner as in Example 1, and the amount of LDH in the culture medium was measured 9 hours after the addition.

In the present invention, primary culture neurons were prepared from the cerebral cortex of rat embryos (17-19-day-old embryos). As a culture medium, MEM (Invitrogen) supplemented with glucose (1 g /L), penici./strept. (100 unit/mL) and 10% FCS was used. The medium was exchanged in every 2 to 3 days. When KA toxicity was examined, Neurobasal Medium (Invitrogen) supplemented with B-27 and glutamine (0.25 mM) was used. Cells were cultured at 37°C under 5% CO₂, After culturing for 7 days or more, 100 µM of NMDA was added to the medium. Then, cells were cultured overnight at 37°C under 5% CO₂. Donepezil was added 12 hours before the addition of NMDA stimulation. Nine hours after the addition of NMDA, the amount of LDH in the culture medium was measured and used as an indicator for nerve protective effect.

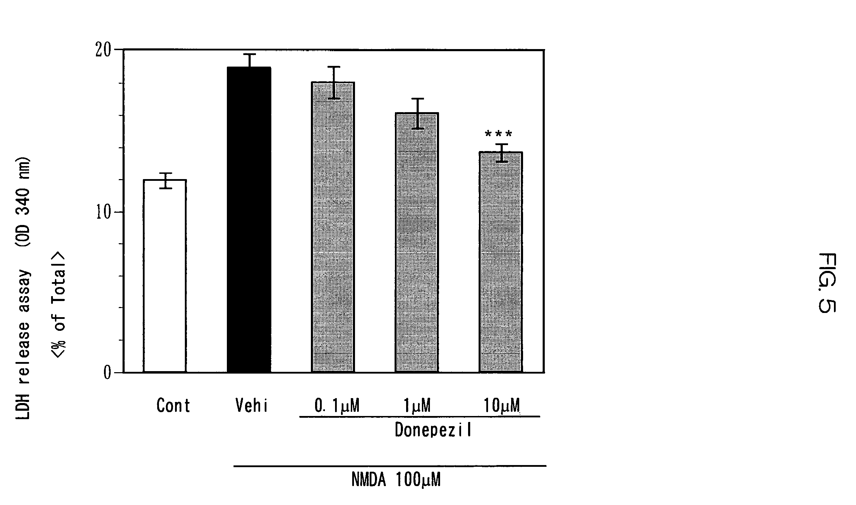

The results of this Example are shown in Fig. 5. From Fig. 5, it is understood that donepezil inhibits the LDH release of neurons in a dose dependent manner in a range from 0.1 µM to 10 µM. According to this experiment, it has become clear that donepezil shows neurocyte protective effect against NMDA excitotoxicity in a dose dependent manner.

### EXAMPLE 3: Excitotoxicity Test

It is said that kainic acid enhances the death of neurons induced by β-amyloid (Aβ) which is said one of the causative factors of Alzheimer's disease. In this Example, a model is used in which cytotoxicity is induced in rat primary culture cerebral cortex neurons by addition of kainic acid.

In the present invention, primary culture neurons were prepared from the cerebral cortex of rat embryos (17-19-day-old embryos). Cells were cultured at 37°C under 5% CO₂. After culturing for 7 days or more, NMDA or kainic acid was added to the medium. Then, cells were cultured overnight at 37°C under 5% CO₂. Donepezil was added 24 hours before the addition of kainic acid stimulation. Twenty-four hours after the addition of kainic acid, the amount of LDH in the culture medium was measured and used as an indicator for nerve protective effect.

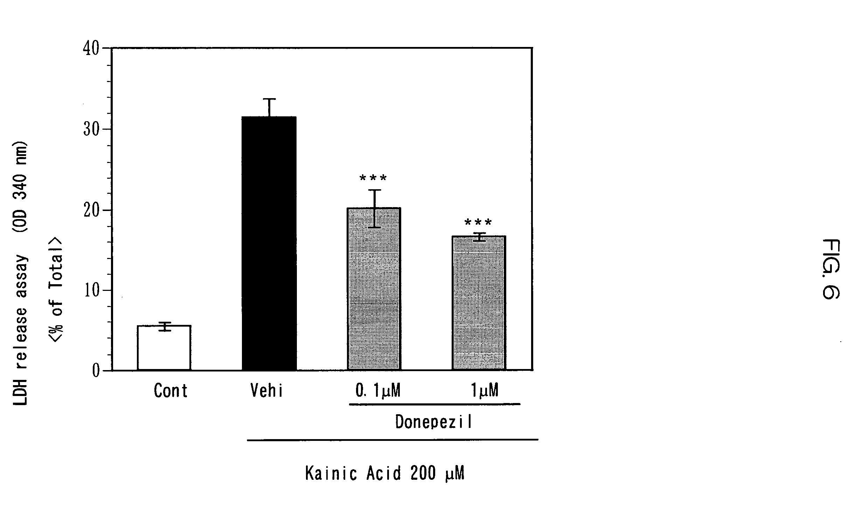

The results of this Example are shown in Fig. 6. From Fig. 6, it is understood that donepezil inhibits the LDH release of neurons in a dose dependent manner in a range from 0.1 µM to 1 µM. According to this experiment, it has become clear that donepezil shows neurocyte protective effect against kainic acid excitotoxicity in a dose dependent manner.

The reagents and statistical analysis methods used in the following Examples 4 to 7 are as described below. Aβ (human, 1-40) was purchased from Peptide Institute, Inc. (Osaka, Japan). Trypsin solution, penicillin, streptomycin, Dulbecco's modified Eagle's medium (DMEM) and HEPES were purchased from Life Technologies Inc. (Grand Island, NY). Insulin, sodium selenite, putrescine, DNase I, mecamylamine and scopolamine were purchased from Sigma Chemical Co. (St. Louis, MO). Fetal calf serum and heat-inactivated horse serum were purchased from Nichirei Co. (Tokyo, Japan). ChAT and microtubule associated protein-2 (MAP2) were purchased from Chemicon International Inc. (Temecula, CA). Alexa Fluor 546 was purchased from Molecular Probes (Eugene, OR). Vecstain Elite ABC kit and diaminobenzidine (DAB) kit were purchased from Vector Laboratories, Inc. (Burlingame, CA). Lipofectamine 2000 was purchased from Invitrogen Co. (Carlsbad, CA). siRNA was synthesized by Japan BioService (Saitama, Japan).

Statistical data are shown as mean ± standard error. For comparative test, Welch's test was used. p<0.05 was regarded as significantly different. The statistical analysis software used was SAS 8.1 (SAS Institute Japan Ltd., Tokyo, Japan).

### EXAMPLE 4: Culture of Septal Area Neurons and Confirmation of Their Being Cholinergic Nerves

Septal area neurons were prepared from embryos (18-day-old embryos) of Wistar rats (Charles River Japan). The septal region (including septal area and basal forebrain) was cut out from embryonic brain and incubated in Ca²⁺/Mg²⁺-free Hanks' balanced salt solution containing 0.25% trypsin and 0.2 mg/ml DNase I at 37°C for 15 minutes. To the resultant cells, 10 % fetal calf serum and 10 % horse serum-added DMEM (containing 5 µg/ml insulin, 30 nmol/L sodium selenite, 100 µmol/L putrecine, 20 nmol/L progesterone, 15 nmol/L biotin, 100 units/ml penicillin, 100 µg/ml streptomycin and 1 mmol/L sodium pyruvate) was added to deactivate trypsin, and then cells were washed twice. Cells were plated on 96-well poly-D-lysine-coated culture plates at a concentration of 1.2 x 10⁵ cells/well. The plated cells were cultured in a CO₂ incubator (5% CO₂ 37°C). After one day, 2/3 of the medium was exchanged for the above-described DMEM containing 5 % fetal calf serum. After three days, 2/3 of the medium was exchanged again for the above-described DMEM containing 5 % fetal calf serum. After 6 days, the entire medium was exchanged for the above-described DMEM (this time, Gln is not contained) not containing serum.

The thus cultured septal area neurons were immunostained with anti-choline acetyltransferase (ChAT) antibody. First, the cultured septal area neurons were fixed in a neutral formalin solution for 60 minutes. After 30 minute incubation with 5 % goat serum, the cells were incubated with a 500-fold dilution of ChAT antibody in the presence of 1% goat serum for one day. Subsequently, the cells were incubated with a 500-fold dilution of goat anti-rabbit IgG and HRP-labeled avidin-biotin complex for one hour, followed by staining with DAB as the substrate for peroxidase.

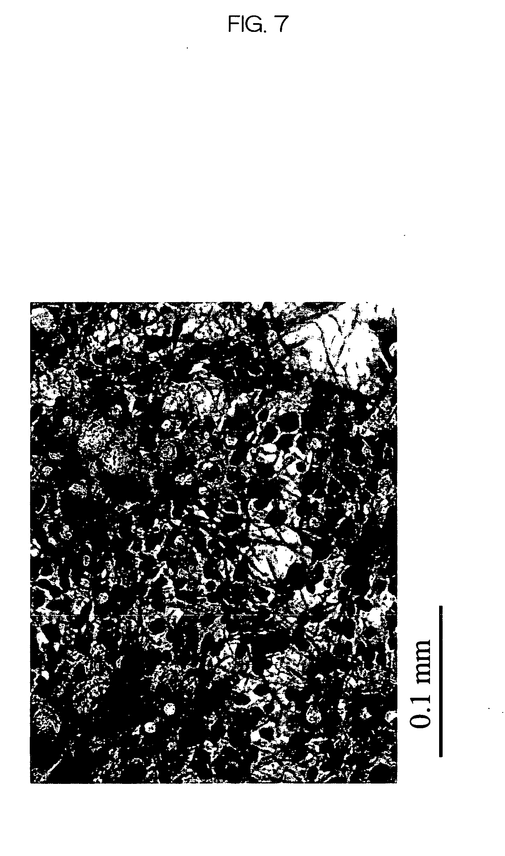

The results of the immunostaining of cultured rat septal area neurons with anti-ChAT antibody are shown in Fig. 7. The scale bar in Fig. 7 represents 0.1 mm. The immunostaining revealed that a great number of the cultured rat septal area neurons have choline acetyltransferase (ChAT) (an acetylcholine synthesis enzyme). This means that the cultured rat septal area neurons are cholinergic nerves. Thus, it could be confirmed that the cells are cholinergic nerves.

It has been reported that the decrease of choline acetyltransferase in the brains of Alzheimer patients correlates to the severity of cognitive disorders (Perry E.K., Tomlinson B.E., Blessed G, Bergman K., Gibson P.H. and Perry R.H. (1978) Correlation of cholinergic abnormalities with senile plaques and mental test scores in senile dementia. Br. Med. J. 2, 1457-1459). Therefore, it can be said that the inhibition of disorders in septal area neurons leads to inhibition of the lowering of cognitive function in Alzheimer patients.

### EXAMPLE 5: Protective Effect of Donepezil against Aβ(1-40) Toxicity and Cell Death Experiment

As an indicator for cytotoxicity, LDH (lactate dehydrogenase) was measured. As cells, septal area neurons cultured for 7 days according to the culture method in Example 4 were used. Briefly, 15 µmol/L of Aβ (1-40) was added to the medium of septal area neurons, and the cells were cultured in a CO₂ incubator (5% CO₂, 37°C). Donepezil (0.01, 0.1, 1, 10 µmol/L), mecamylamine (10 µmol/L) and scopolamine (10 µmol/L) were added 24 hours before the addition of Aβ(1-40). Forty-eight hours after the addition of Aβ(1-40), lactate dehydrogenase (LDH) activity in the medium was measured. At the same time, cells were solubilized with phosphate buffer (pH 7.4) containing 0.5% Triton X-100, followed by measurement of LDH in cells. Out of the total LDH (present in the cells and in the medium), the amount of LDH which leaked out into the medium was determined and expressed in % (Koh J.Y. and Choi D.W. (1987) Quantitative determination of glutamate mediated cortical neuronal injury in cell culture by lactate dehydrogenase efflux assay. J. Neurosci. Meth. 20, 83-90). The data were analyzed by analysis techniques of ANOVA and Welch's test.

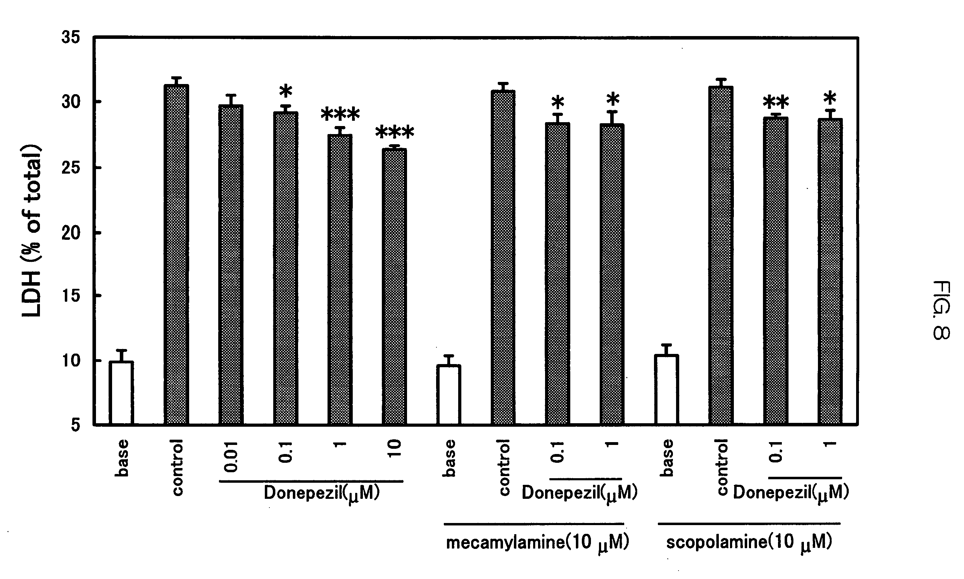

The results are shown in Fig. 8. In Fig. 8, open columns represent cells to which no Aβ was added; and gray columns represent cells to which Aβ was added. Individual LDH values were determined as mean ± S.E.M. (n=23-25) and summarized in a graph. In Fig. 8, mark " * " represents p<0.05; mark " ** " represents p<0.01; and mark " *** " represents p<0.001 ("control cells" was used as control).

The cultured septal area neurons were susceptible to disorders by the addition of Aβ(1-40) (see "control cells" in Fig. 8). Donepezil showed significant protective effect from 0.1 µmol/L against the toxicity when Aβ(1-40) was added for 48 hours (Fig. 8). The inhibitory effect of donepezil against Aβ(1-40) toxicity was not inhibited by acetylcholine receptor antagonists mecamylamine and scopolamine (Fig. 8).

Subsequently, the cultured rat septal area neurons treated with Aβ(1-40) in the same manner as described above were immunostained with anti-MAP2 antibody. MAP2 is used as a neurocyte marker (Herzog W. and Weber K. (1978) Fractionation of brain microtubule-associated proteins. Isolation of two different proteins which stimulate tubulin polymerization in vitro. Eur J Biochem. 92(1), 1-8). MAP2 staining was performed based on the immunostaining method described in Example 4 above. Briefly, 48 hours after the addition of 15 µmol/L of Aβ(1-40) to the cells, the cells were fixed and immunostained. Donepezil was added in advance 24 hours before the addition of Aβ(1-40). After fixation, the cells were incubated with a 300-fold dilution of anti-MAP2 antibody for one day, followed by detection using Alexa Fluor546 as a fluorescent substrate.

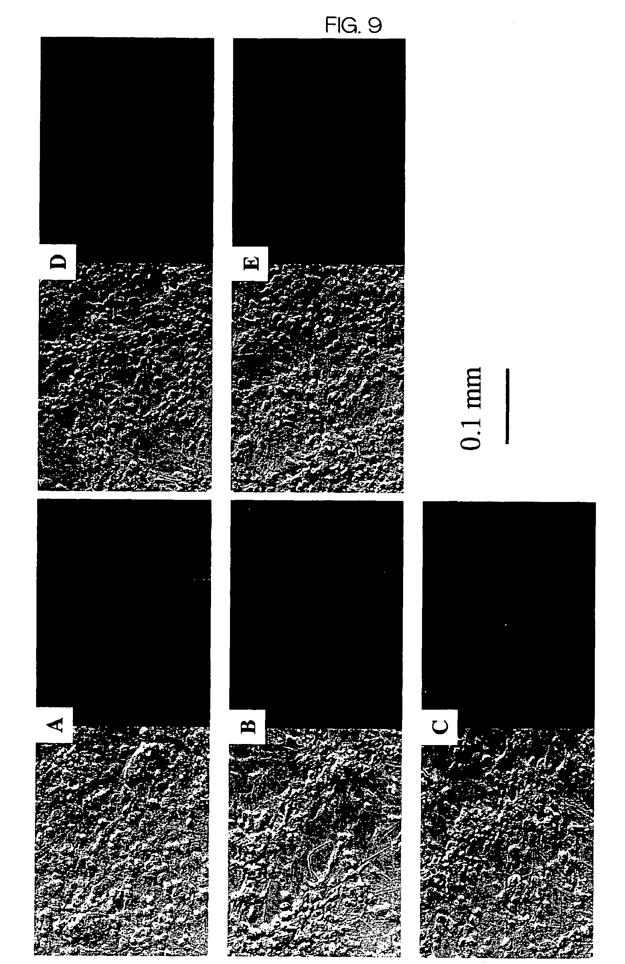

The results are shown in Fig. 9. The scale bar represents 0.1 mm. Panel A shows control cells to which Aβ(1-40) was not added. Panel B shows cells to which Aβ(1-40) was added. Panels C, D and E show cells to which a combination of Aβ(1-40) and donepezil (0.1, 1 and 10 µmol/L) was added. The photograph at the left in each panel is a bright field photograph by Hoffman modulation. The photograph at the right in each panel is a photograph of fluorescence. When Aβ(1-40) was added for 48 hours, the number of neurons stained with MAP2 decreased (Fig. 9B). It was recognized that donepezil inhibits the disorders in MAP2-stained cells induced by Aβ(1-40) (Fig. 9).

### EXAMPLE 6: Effect of Donepezil on Aβ(1-40) Aggregation

In order to examine the structural state of Aβ(1-40) in culture medium, CD spectra were measured. It is known that when Aβ takes a β-sheet structure, Aβ readily forms mass of Aβfiber and shows toxicity (Howlett D.R., Jennings K.H., Lee D.C., Clark M.S., Brown F., Wetzel R., Wood S.J., Camilleri P. and Roberts GW. (1995) Aggregation state and neurotoxic properties of Alzheimer beta-amyloid peptide. *Neurodegeneration.* 4, 23-32). It is also known that CD (circular dichroism) spectra at 205-225 nm decrease when Aβ(1-49) forms an a-helix structure or a β-sheet structure (Sreerama N., Venyaminov S.Y. and Woody R.W. (2000) Estimation of peptide secondary structure from circular dichroism spectra: inclusion of denatured peptides with native peptides in the analysis. Anal. Biochem. 287, 243-251; Bokvist M., Lindstrom F., Watts A. and Grobner G (2004) Two types of Alzheimer's beta-amyloid (1-40) peptide membrane interactions: aggregation preventing transmembrane anchoring versus accelerated surface fibril formation. J. Mol. Biol. 335, 1039-49). In particular, it is known that the formation of a β-sheet structure decreases CD spectra around 215 nm. Then, CD was measured as an indicator for Aβ aggregation.

Briefly, 15 µmol/L of Aβ(1-40) was added to cultured septal area neurons. Forty-eight hours after the addition, medium samples were taken, followed by measurement of CD spectra at 215-260 nm. A JASCO spectrometer model J-720WI (JASCO Corporation) was used for the measurement.

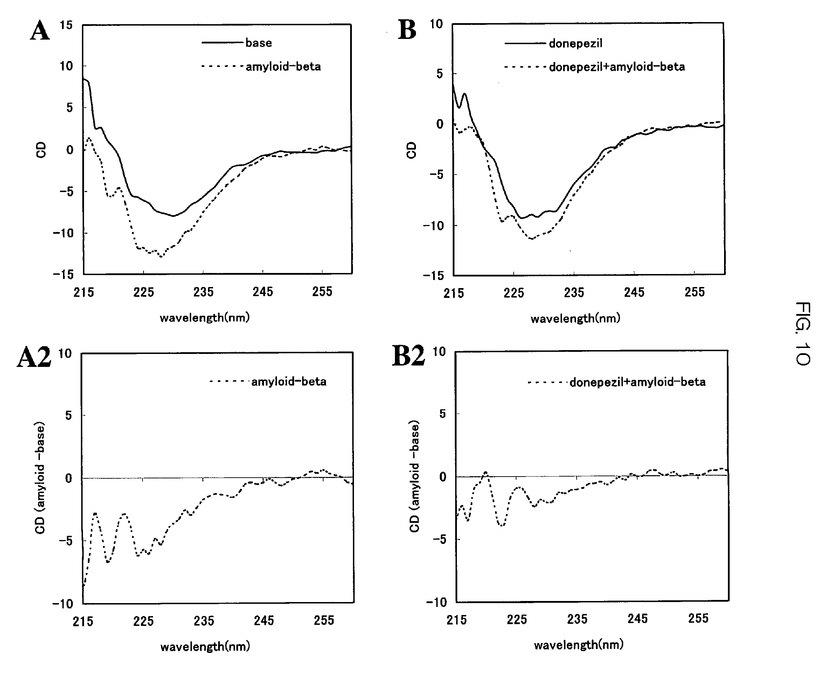

The results are shown in Fig. 10. Panel A shows cells to which donepezil was not added. Panel B shows cells to which donepezil (10 µM) was added. Solid line represents cells to which Aβ was not added. Dotted line represents cells to which Aβ was added. Panels A2 and B2 show graphs obtained by subtracting the values of the solid lines from the values of the dotted lines in graphs A and B, respectively. The decrease of CD spectra at 215-225 nm was inhibited by the addition of 10 µmol/Lof donepezil. Therefore, according to this Example, it was demonstrated that donepezil inhibits the formation of a-helix structure or β-sheet structure by Aβ.

### EXAMPLE 7: Effect of siRNA on Intracellular Acetylcholine Activity and Its Protective Effect against Aβ(1-40) Toxicity

### (1) siRNA Treatment

The following two sets of oligonucleotides [(i) and (ii)] were used as the double-stranded siRNAs of acetyl cholinesterase.
(i) ; 5'-AAUGUCAGUGACUCUGUUUTT-3' (SEQ ID NO: 1) 5'-AAACAGAGUCACUGACAUUTT-3'    (SEQ ID NO: 2) and
(ii) ;

5'-GCUGCCUCAAGAAAGUAUCTT-3'    (SEQ ID NO: 3)

5'-GAUACUUUCUUGAGGCAGCTT-3'     (SEQ ID NO: 4)

On day 6 and 7 of culture, 1 µmol/L of siRNA (i) and siRNA (ii) above and lipofectamine 2000 (0.4 µL/mL) were added to the medium of septal area neurons. On day 9 of culture, cells were solubilized and intracellular acetylcholinesterase activity (AChE activity) was measured.

### (2) Measurement of Acetylcholinesterase Activity

Measurement of acetylcholinesterasesterase activity was performed according to the method of Ellman et al. (Ellman GL., Courtney K.D., Anders VJ., Feather-Stone R.M. (1961) A new and rapid colorimetric determination of acetylcholinesterase activity. Biochem. Pharmacol. 7, 88-95). Briefly, cells were solubilized in 0.5 % Triton X-100-containing phosphate buffer (pH 8.0). The resultant solubilized cell solution was centrifuged, and the supernatant was diluted 3-fold with phosphate buffer (pH 8.0). To this diluted supernatant, 5,5'-dithiobis(2-nitrobenzoic acid) (0.33 mmol/L) and acetylthiocholine iodide (AthCh) (0.5 mmol/L) were added and agitated. Then, increase in absorbance at 412 nm was measured (Molecular Devices, SpectraMax 190EXT). The data were analyzed by ANOVA and Welch's test.

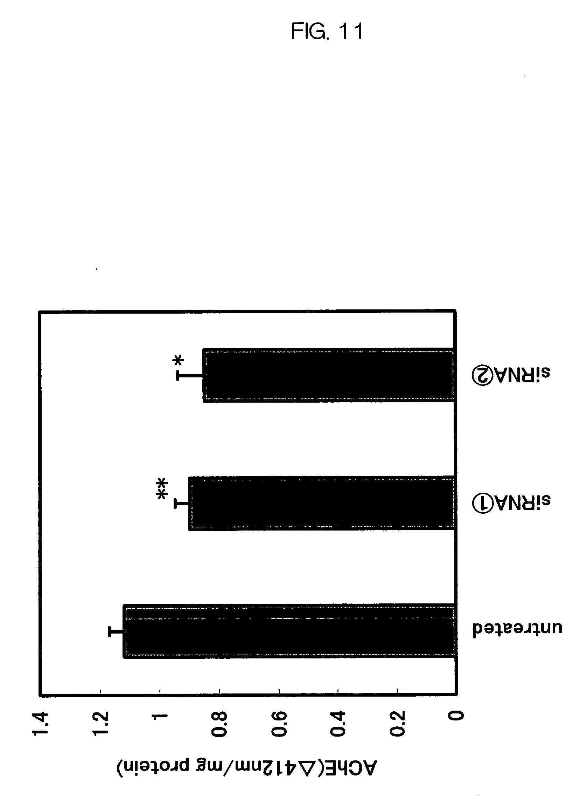

The results are shown in Fig. 11. Individual values were obtained as mean ± S.E.M. (n=11) and summarized in a graph (n=11). In Fig. 11, mark " ** " represents p<0.01 and mark " *** " represents p<0.001, relative to the cells which were not treated with siRNA.

As a result of examination of two siRNAs (i) and (ii), it was recognized that both siRNAs have AChE activity inhibitory effect (Fig. 11).

### (3) Protective Effect of siRNA against Aβ(1-40) Toxicity

LDH (lactate dehydrogenase) was measured as an indicator for cytotoxicity. Briefly, 15 µmol/L of Aβ(1-40) was added to cultured septal area neurons on day 7 of culture and incubated for 48 hours. siRNA (1 µmol/L) was added 24 hours before and immediately before the addition of Aβ(1-40). Open columns represent cells to which Aβ(1-40) was not added. Gray columns represent cells to which Aβ(1-40) was added. The data were analyzed by ANOVA and Welch's test.

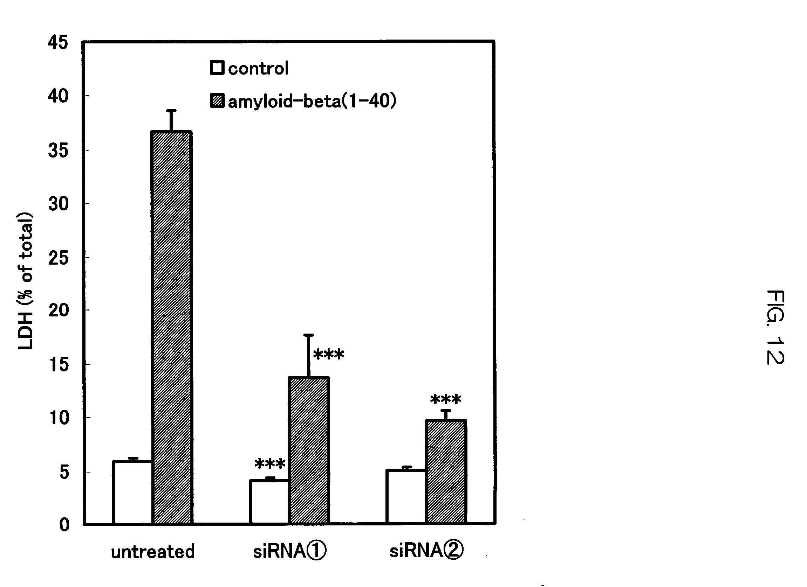

The results are shown in Fig. 12. Individual values were obtained as mean ± S.E.M. and summarized in a graph (n=7). In Fig. 12, mark " *** " represents p<0.001, relative to the cells which were not treated with siRNA.

As a result of examination of two siRNAs (i) and (ii), it was found that both siRNAs inhibit Aβ toxicity (Fig. 12).

In parallel with the measurement of LDH, Aβ aggregation was analyzed with changes in CD spectra at 215-260 nm.

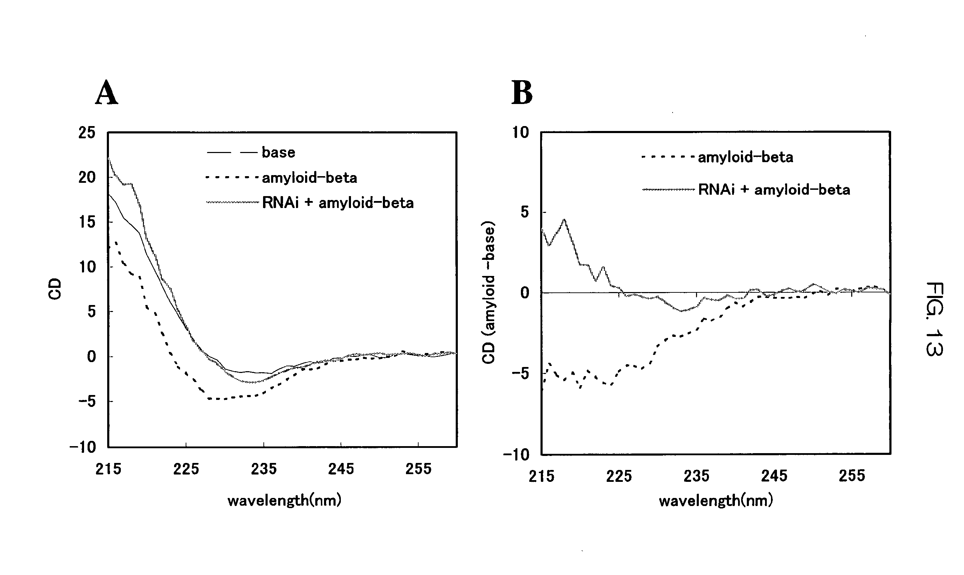

The results are shown in Fig. 13. Panel A shows cells to which RNAi was added. Solid line represents cells to which Aβ was not added. Dotted line represents cells to which Aβ was added. Panel B shows the values obtained by subtracting the values of dotted line from the values of solid line. Decrease in CD spectra at 215-225 nm was inhibited by the addition of siRNA (Fig. 13).

### INDUSTRIAL APPLICABILITY

The neurocyte protective agent of the present invention works in a neurocyte protective manner against ischemia-like cytotoxicity and injury induced by excitotoxicity. Therefore, with the neurocyte protective agent of the present invention, it is possible to prevent or inhibit the ischemic death of neurons induced by cerebral infarction, cerebral thrombosis, or the like and the death of neurons induced by excitotoxicity.

### SEQUENCE LISTING FREE TEXT

SEQ ID NO: 1: siRNA
SEQ ID NO: 2: siRNA
SEQ ID NO: 3: siRNA
SEQ ID NO: 4: siRNA

## Claims

1. A protective agent for neurons of the central nervous system, comprising any one of the compounds shown in the following (i) to (vii):
(i) 1-benzyl-4-[(5,6-dimethoxy-1-indanone)-2-yl]methylpiperidine represented by the following chemical formula or a pharmacologically acceptable salt thereof: 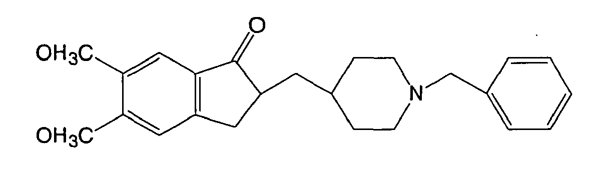
(ii) a cyclic amine derivative represented by the following general formula (I) or a pharmacologically acceptable salt thereof: 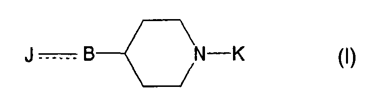
where J is a monovalent or divalent group selected from the groups represented by the following formulas: 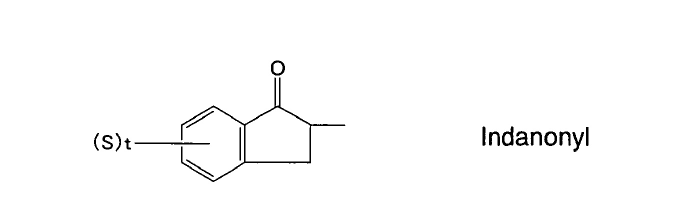 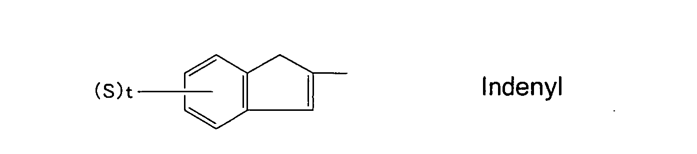 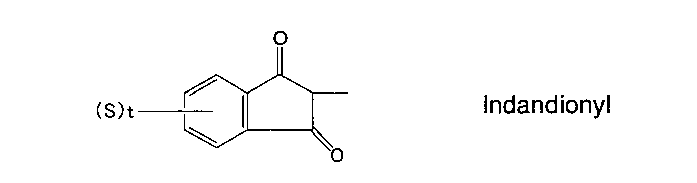 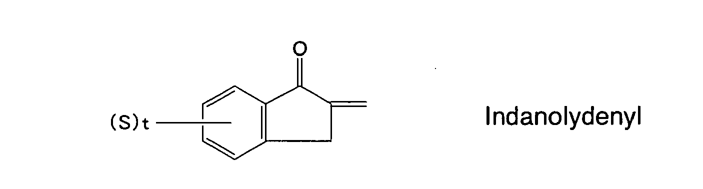 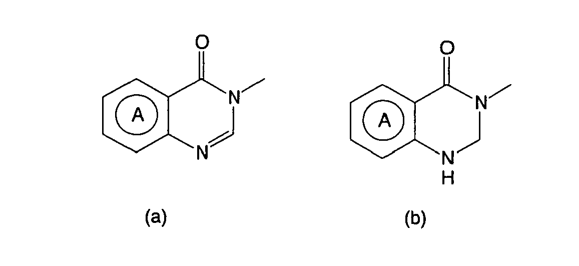 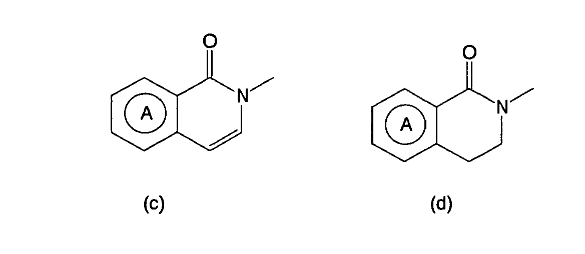 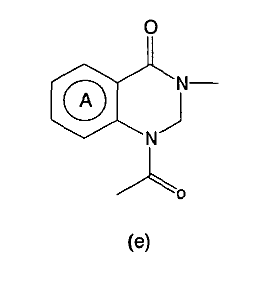 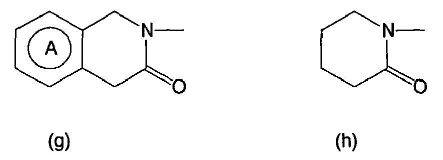 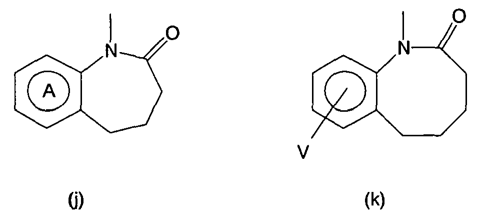 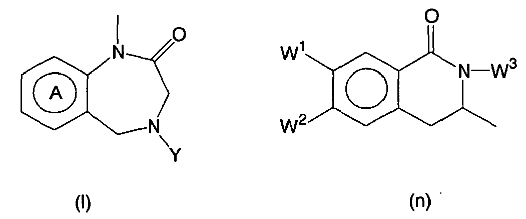 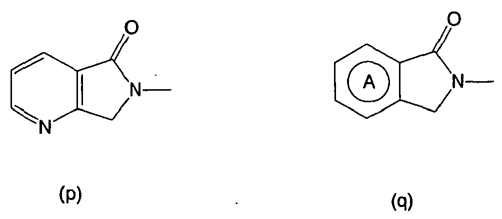
where S is a lower alkyl group with 1 to 6 carbon atoms, a lower alkoxy group with 1 to 6 carbon groups, a halogen atom or a hydroxyl group; t is 0 or an integer from 1 to 4; (S)ₜ may form a methylenedioxy or ethylenedioxy group between adjacent carbon atoms on the phenyl ring linked; Y in formula (1) is a hydrogen atom or a lower alkyl group with 1 to 6 carbon atoms; V in formula (k) is a hydrogen atom or a lower alkoxy group with 1 to 6 carbon atoms; W¹ and W² in formula (n) independently represent, similarly or differently, a hydrogen atom, a lower alkyl group with 1 to 6 carbon atoms, or a lower alkoxy group with 1 to 6 carbon atoms; W³ in formula (n) is a hydrogen atom or a lower alkyl group with 1 to 6 carbon atoms; phenyl ring A in formulas (a) to (e), (g), (j), (l) and (q) may be substituted with an alkyl group with 1 to 6 carbon atoms or a alkoxy group with 1 to 6 carbon atoms;
B is a group represented by a formula -(CHR²)ₙ- (where n is 0 or an integer from 1 to 10; R² is each independently a hydrogen atom or a methyl group), a group represented by a formula =(CH-CH=CH)_{b}- (where b is an integer from 1 to 3), a group represented by a formula =CH-(CH₂)_{c}- (where c is 0 or an integer from 1 to 9), or a group represented by a formula =(CH-CH)_{d}= (where d is 0 or an integer from 1 to 5);
K is a phenylalkyl group that may have, as a substituent, an alkyl group with 1 to 6 carbon atoms which may be halogenated, an alkoxy group with 1 to 6 carbon atoms, a nitro group, a halogen atom, a carboxyl group, a benzyloxy group, an alkoxycarbonyl group with 1 to 6 carbon atoms, an amino group, a monoalkylamino group with 1 to 6 carbon atoms, a dialkylamino group with 1 to 6 carbon atoms, a carbamoyl group, an acylamino group with 1 to 6 carbon atoms, a cyclohexyloxycarbonyl group, an alkylaminocarbonyl group with 1 to 6 carbon atoms, an alkylcarbonyloxy group with 1 to 6 carbon atoms, a hydroxyl group, a formyl group or an alkoxy (with 1 to 6 carbon atoms)-alkyl (with 1 to 6 carbon atoms) group; and 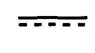 represents a single bond or a double bond;
(iii) a cyclic amine derivative selected from the compounds represented by the following formulas or a pharmacologically acceptable salt thereof: 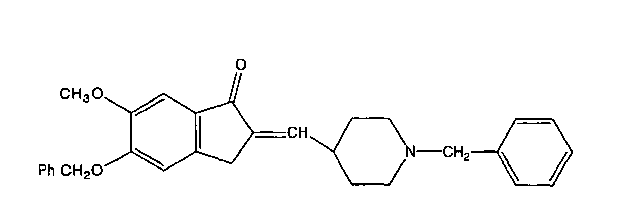 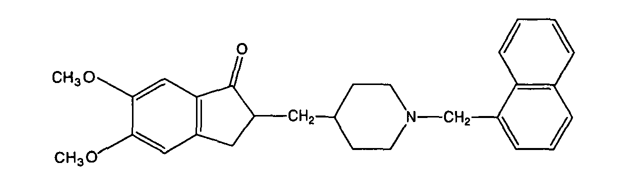 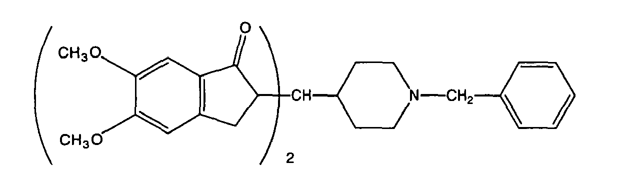 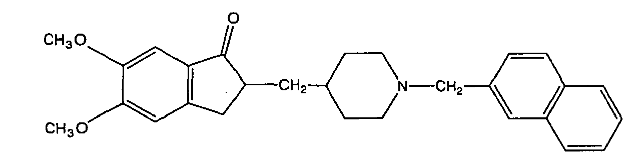 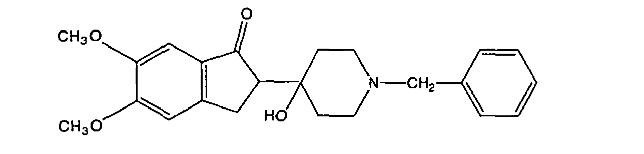 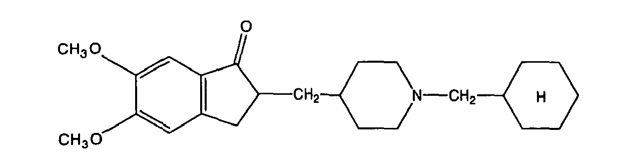 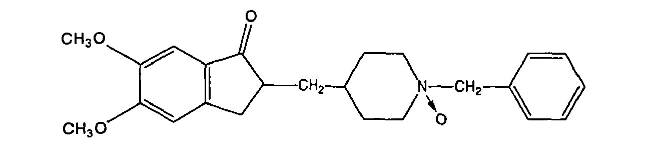
(iv) a cyclic amine derivative selected from the compounds represented by the following formulas or a pharmacologically acceptable salt thereof: 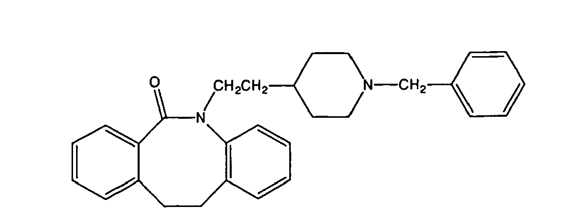 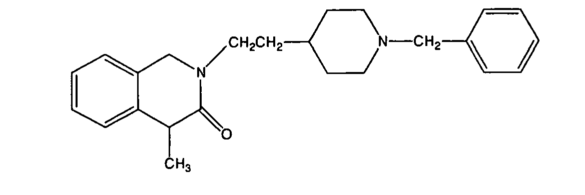 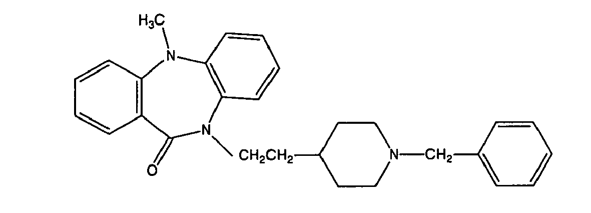 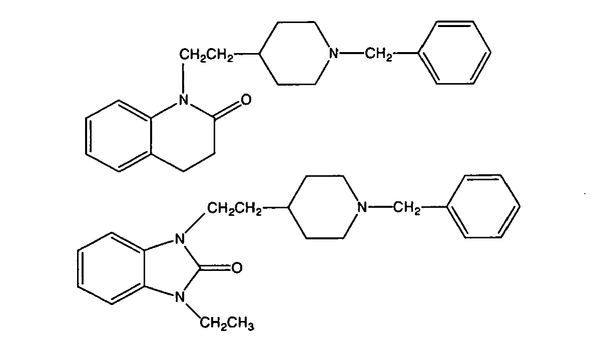 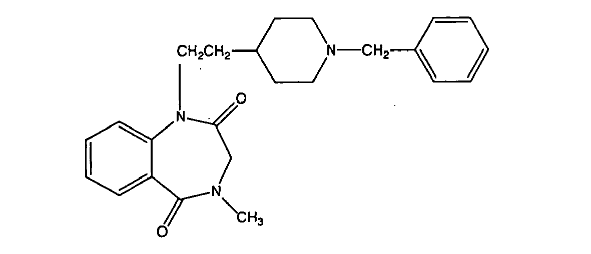 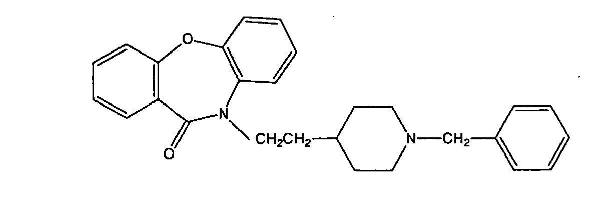
(v) a cyclic amine derivative represented by the following general formula (I-1) or a pharmacologically acceptable salt thereof: 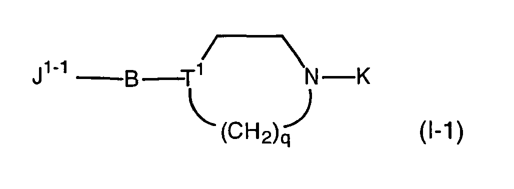 where J^{I-1} is a lower alkyl group with 1 to 6 carbon atoms (hereinafter, just referred to as "lower alkyl group"); a cyclohexyl group; a phenyl, pyridyl or pyrazyl group which may have, as a substituent, a lower alkyl group, a lower alkoxy group with 1 to 6 carbon atoms (hereinafter, just referred to as "lower alkoxy group"), a nitro group, a halogen, a carboxyl group, a lower alkoxycarbonyl group, an amino group, a mono-lower alkylamino group, a di-lower alkylamino group, a carbamoyl group, an acylamino group derived from aliphatic saturated monocarboxylic acid with 1 to 6 carbon atoms, a cyclohexyloxycarbonyl group, a lower alkylaminocarbonyl group, a lower alkylcarbonyloxy group, a halogenated lower alkyl group, a hydroxyl group, a formyl group or a lower-alkoxy-lower-alkyl group; a group represented by a formula 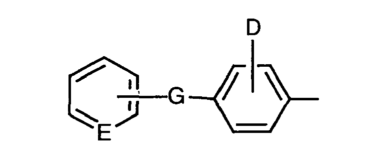 where G is a group represented by a formula 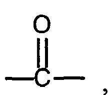 a group represented by a formula 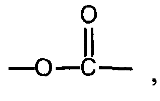 a group represented by a formula -O- , a group represented by a formula 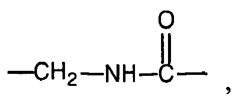 a group represented by a formula -CH₂-O-, a group represented by a formula -CH₂-SO₂- , a group represented by a formula 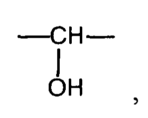 or a group represented by a formula 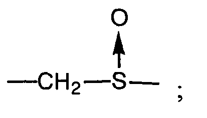 E is a carbon atom or a nitrogen atom;
a quinolyl group; a quinoxalyl group; a furyl group or a group represented by a formula R¹-CH=CH- (where R¹ is a hydrogen atom or a lower alkoxycarbonyl group);
B is a group represented by a formula -(CH₂)ₙ-, a group represented by a formula -NR²-(CH₂)ₙ- (where R² is a hydrogen atom, a lower alkyl group, a phenyl group or a lower alkylsulfonyl group), a group represented by a formula -CONR³-(CH₂)ₙ- (where R³ is a hydrogen atom, a lower alkyl group, a phenyl, benzyl or pyridyl group which may have, as a substituent, a lower alkyl group, a lower alkoxy group, a halogen or a hydroxyl group), a group represented by a formula -NH-CO-(CH₂)ₙ-, a group represented by a formula -CH₂-CO-NH-(CH₂)ₙ-, a group represented by a formula -CO-CH₂-CH(OH)-CH₂-, a group represented by a formula -CO-(CH₂)ₙ-, a group represented by a formula -C(OH)-(CH₂)ₙ- or a group represented by a formula -CO-CH=CH-CH₂-; and n in the above formulas is 0 or an integer from 1 to 10;
T¹ is a carbon atom;
K is a phenylalkyl group (where the alkyl has 1 to 2 carbon atoms) in which the phenyl may have, as a substituent, a lower alkyl group, a lower alkoxy group, a nitro group, a halogen, a carboxyl group, a lower alkoxycarbonyl group, an amino group, a mono-lower alkylamino group, a di-lower alkylamino group, a carbamoyl group, an acylamino group derived from aliphatic saturated monocarboxylic acid with 1 to 6 carbon atoms, a cyclohexyloxycarbonyl group, a lower alkylaminocarbonyl group, a lower alkylcarbonyloxy group, a halogenated lower alkyl group, a hydroxyl group, a formyl group or a lower-alkoxy-lower-alkyl group; a cinnamyl group; a lower alkyl group; a pyridyl methyl group; a cycloalkyl (with 3 to 6 carbon atoms)-alkyl group; an adamantanemethyl group; a furfuryl group; a cycloalkyl group with 3 to 6 carbon atoms; or an acyl group; and
q is 1 or 2;
(vi) a cyclic amine derivative represented by the following general formula (I-2) or a pharmacologically acceptable salt thereof: 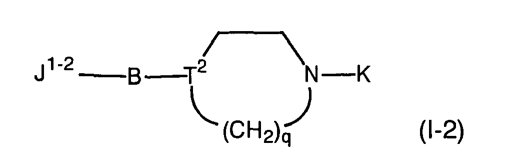
where J¹⁻² is an indanonyl group which may have, as a substituent, a lower alkyl group with 1 to 6 carbon atoms or a lower alkoxy group with 1 to 6 carbon atoms; T² is a nitrogen atom; B, K and q are the same as defined above;
(vii) a cyclic amine derivative selected from the compounds represented by the following formulas or a pharmacologically acceptable salt thereof:

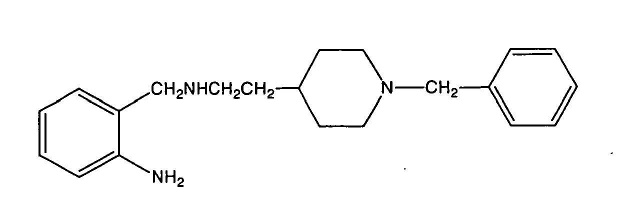

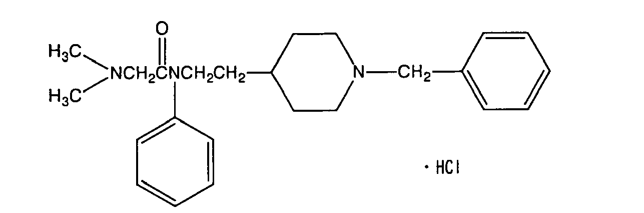

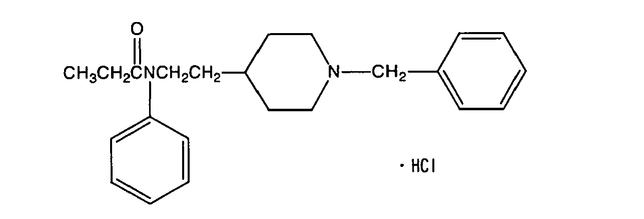

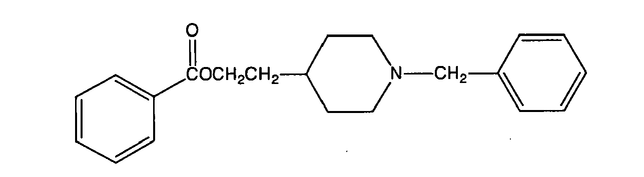

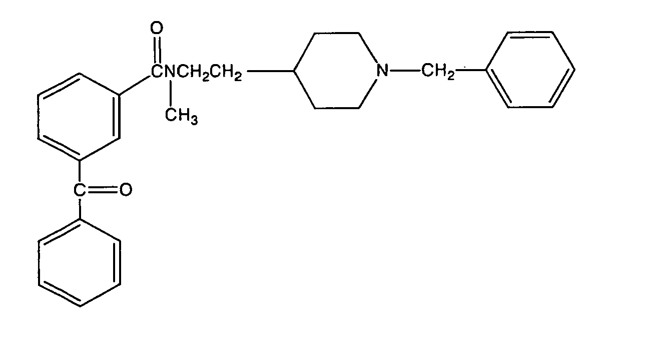

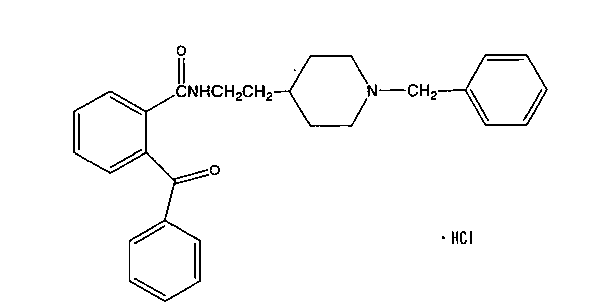

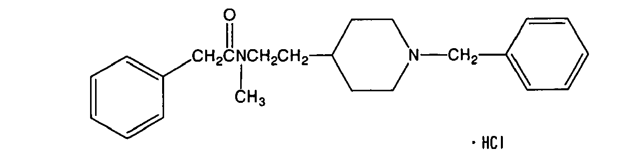

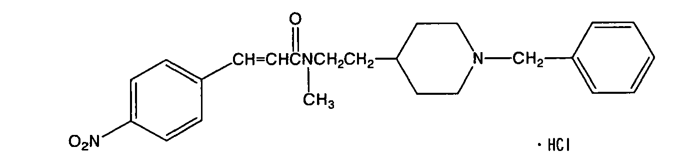

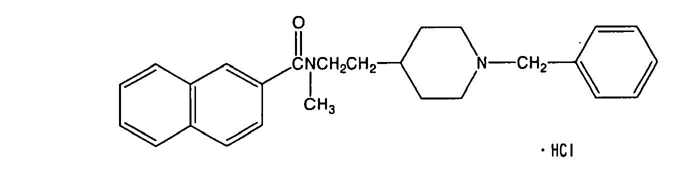

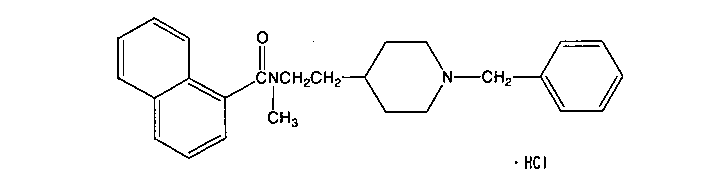

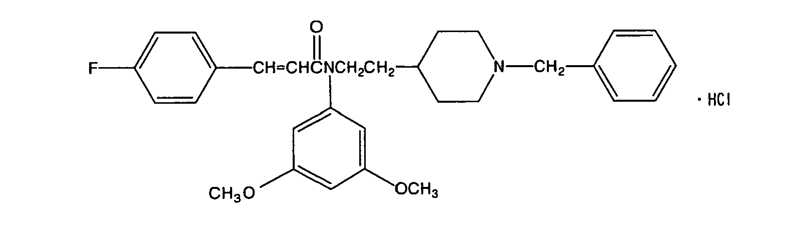

2. The protective agent according to claim 1, wherein the salt is a hydrochloride salt.

3. A prophylactic and/or therapeutic agent for disorders in neurons of the central nervous system, comprising any one of the compounds shown in the following (i) to (vii):
(i) 1-benzyl-4-[(5,6-dimethoxy-1-indanone)-2-yl]methylpiperidine represented by the following chemical formula or a pharmacologically acceptable salt thereof: 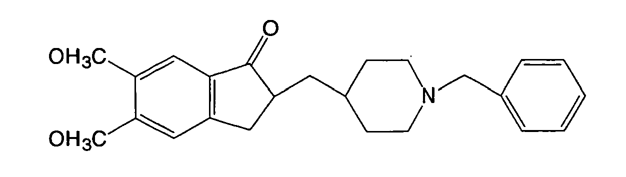
(ii) a cyclic amine derivative represented by the following general formula (I) or a pharmacologically acceptable salt thereof: 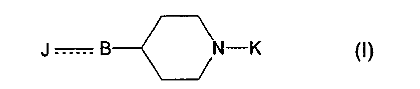 where J is a monovalent or divalent group selected from the groups represented by the following formulas: 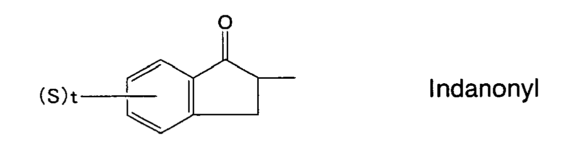 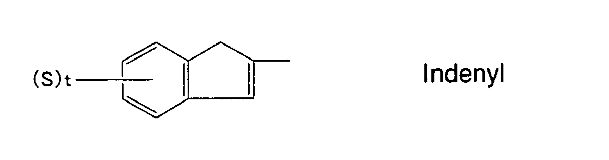 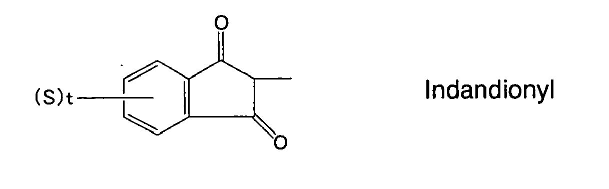 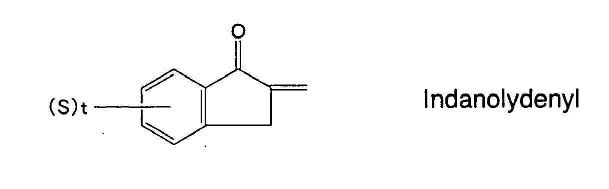 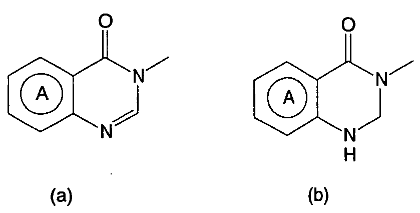 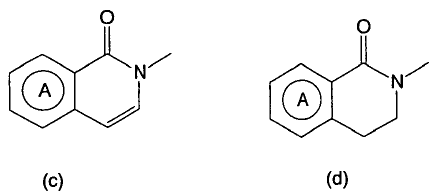 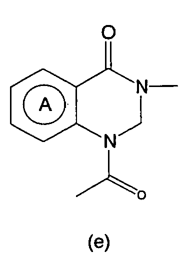 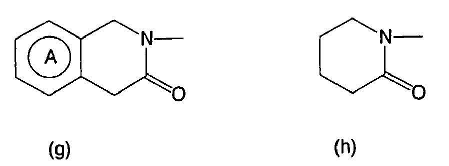 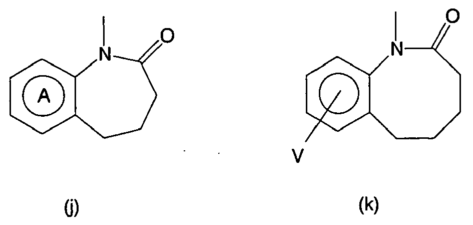 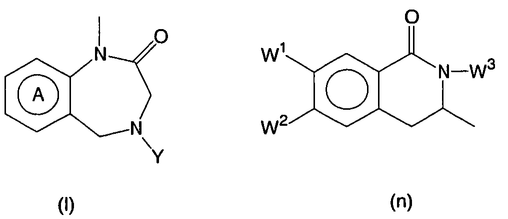 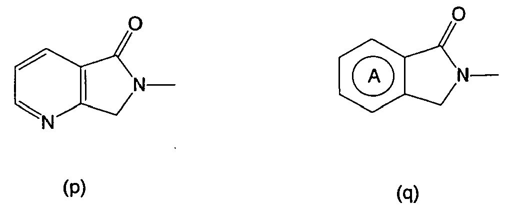
where S is a lower alkyl group with 1 to 6 carbon atoms, a lower alkoxy group with 1 to 6 carbon groups, a halogen atom or a hydroxyl group; t is 0 or an integer from 1 to 4; (S)ₜ may form a methylenedioxy or ethylenedioxy group between adjacent carbon atoms on the phenyl ring linked; Y in formula (1) is a hydrogen atom or a lower alkyl group with 1 to 6 carbon atoms; V in formula (k) is a hydrogen atom or a lower alkoxy group with 1 to 6 carbon atoms; W¹ and W² in formula (n) independently represent, similarly or differently, a hydrogen atom, a lower alkyl group with 1 to 6 carbon atoms, or a lower alkoxy group with 1 to 6 carbon atoms; W³ in formula (n) is a hydrogen atom or a lower alkyl group with 1 to 6 carbon atoms; phenyl ring A in formulas (a) to (e), (g), (j), (l) and (q) may be substituted with an alkyl group with 1 to 6 carbon atoms or a alkoxy group with 1 to 6 carbon atoms;
B is a group represented by a formula -(CHR²)ₙ- (where n is 0 or an integer from 1 to 10; R² is each independently a hydrogen atom or a methyl group), a group represented by a formula =(CH-CH=CH)_{b}- (where b is an integer from 1 to 3), a group represented by a formula =CH-(CH₂)_{c}- (where c is 0 or an integer from 1 to 9), or a group represented by a formula =(CH-CH)_{d}= (where d is 0 or an integer from 1 to 5);
K is a phenylalkyl group that may have, as a substituent, an alkyl group with 1 to 6 carbon atoms which may be halogenated, an alkoxy group with 1 to 6 carbon atoms, a nitro group, a halogen atom, a carboxyl group, a benzyloxy group, an alkoxycarbonyl group with 1 to 6 carbon atoms, an amino group, a monoalkylamino group with 1 to 6 carbon atoms, a dialkylamino group with 1 to 6 carbon atoms, a carbamoyl group, an acylamino group with 1 to 6 carbon atoms, a cyclohexyloxycarbonyl group, an alkylaminocarbonyl group with 1 to 6 carbon atoms, an alkylcarbonyloxy group with 1 to 6 carbon atoms, a hydroxyl group, a formyl group or an alkoxy (with 1 to 6 carbon atoms)-alkyl (with 1 to 6 carbon atoms) group; and 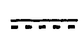 represents a single bond or a double bond;
(iii) a cyclic amine derivative selected from the compounds represented by the following formulas or a pharmacologically acceptable salt thereof: 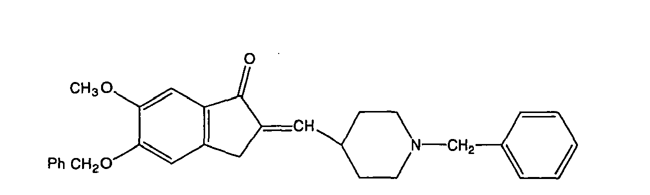 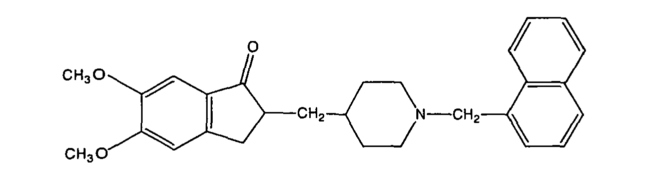 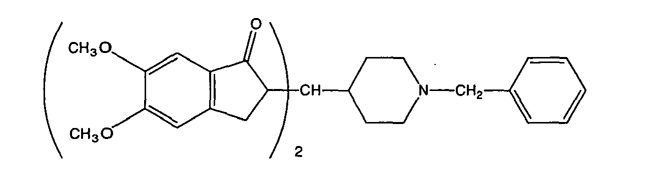 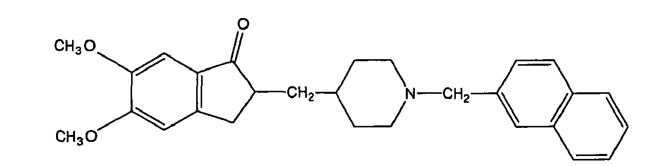 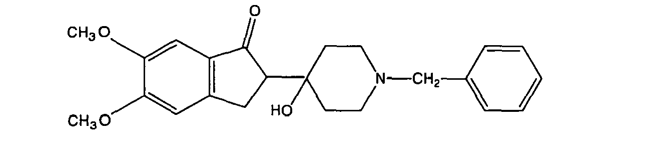 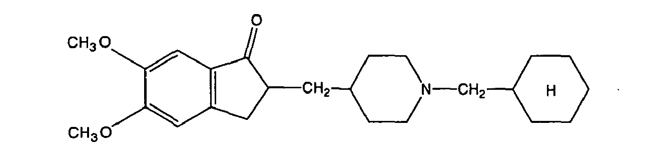 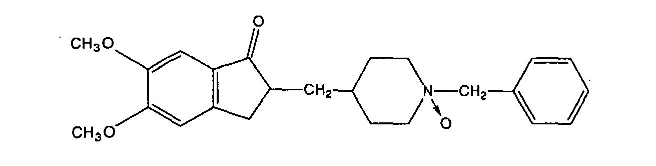
(iv) a cyclic amine derivative selected from the compounds represented by the following formulas or a pharmacologically acceptable salt thereof: 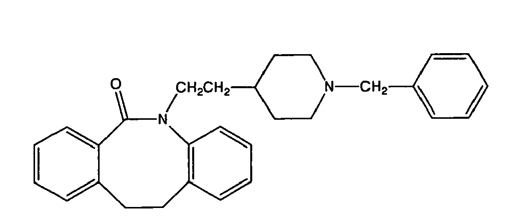 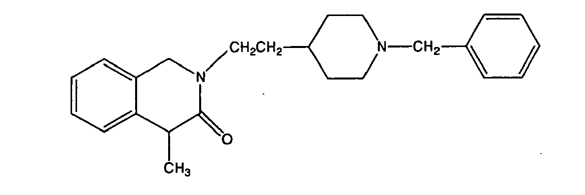 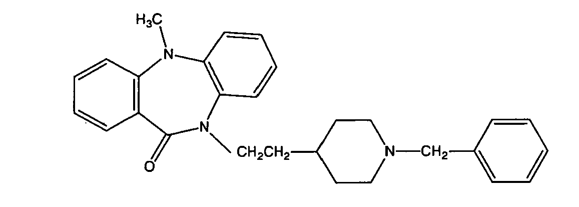 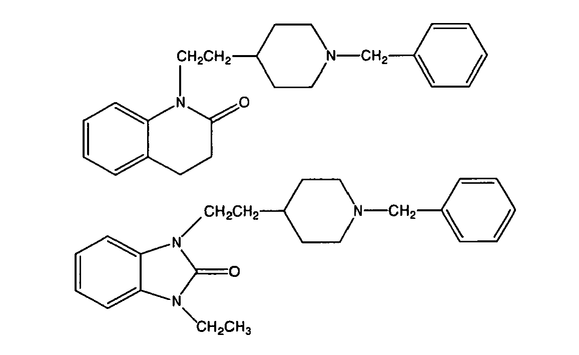 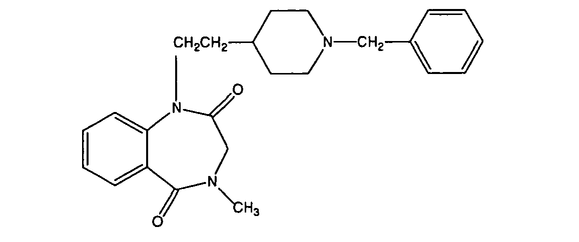 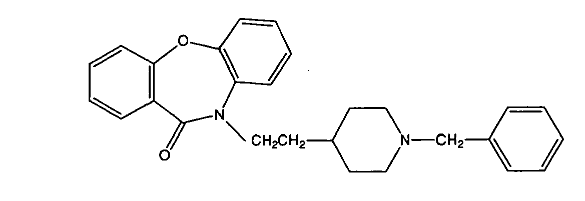
(v) a cyclic amine derivative represented by the following general formula (I-1) or a pharmacologically acceptable salt thereof: 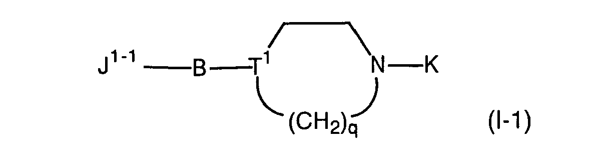 where J¹⁻¹ is a lower alkyl group with 1 to 6 carbon atoms (hereinafter, just referred to as "lower alkyl group"); a cyclohexyl group; a phenyl, pyridyl or pyrazyl group which may have, as a substituent, a lower alkyl group, a lower alkoxy group with 1 to 6 carbon atoms (hereinafter, just referred to as "lower alkoxy group"), a nitro group, a halogen, a carboxyl group, a lower alkoxycarbonyl group, an amino group, a mono-lower alkylamino group, a di-lower alkylamino group, a carbamoyl group, an acylamino group derived from aliphatic saturated monocarboxylic acid with 1 to 6 carbon atoms, a cyclohexyloxycarbonyl group, a lower alkylaminocarbonyl group, a lower alkylcarbonyloxy group, a halogenated lower alkyl group, a hydroxyl group, a formyl group or a lower-alkoxy-lower-alkyl group; a group represented by a formula 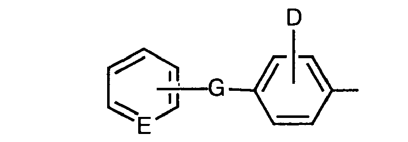 where G is a group represented by a formula 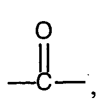 a group represented by a formula 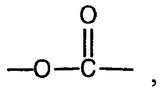 a group represented by a formula -O- , a group represented by a formula 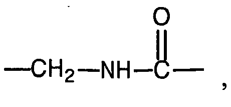 a group represented by a formula -CH₂-O-, a group represented by a formula -CH₂-SO₂ a group represented by a formula 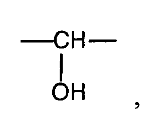 or a group represented by a formula 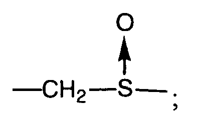 E is a carbon atom or a nitrogen atom; a quinolyl group; a quinoxalyl group; a furyl group or a group represented by a formula R¹-CH=CH- (where R¹ is a hydrogen atom or a lower alkoxycarbonyl group);
B is a group represented by a formula -(CH₂)ₙ-, a group represented by a formula -NR²-(CH₂)ₙ- (where R² is a hydrogen atom, a lower alkyl group, a phenyl group or a lower alkylsulfonyl group), a group represented by a formula -CONR³-(CH₂)ₙ- (where R³ is a hydrogen atom, a lower alkyl group, a phenyl, benzyl or pyridyl group which may have, as a substituent, a lower alkyl group, a lower alkoxy group, a halogen or a hydroxyl group), a group represented by a formula -NH-CO-(CH₂)ₙ-, a group represented by a formula -CH₂-CO-NH-(CH₂)ₙ-, a group represented by a formula -CO-CH₂-CH(OH)-CH₂-, a group represented by a formula -CO-(CH₂)ₙ-, a group represented by a formula -C(OH)-(CH₂)ₙ- or a group represented by a formula -CO-CH=CH-CH₂-; and n in the above formulas is 0 or an integer from 1 to 10;
T¹ is a carbon atom;
K is a phenylalkyl group (where the alkyl has 1 to 2 carbon atoms) in which the phenyl may have, as a substituent, a lower alkyl group, a lower alkoxy group, a nitro group, a halogen, a carboxyl group, a lower alkoxycarbonyl group, an amino group, a mono-lower alkylamino group, a di-lower alkylamino group, a carbamoyl group, an acylamino group derived from aliphatic saturated monocarboxylic acid with 1 to 6 carbon atoms, a cyclohexyloxycarbonyl group, a lower alkylaminocarbonyl group, a lower alkylcarbonyloxy group, a halogenated lower alkyl group, a hydroxyl group, a formyl group or a lower-alkoxy-lower-alkyl group; a cinnamyl group; a lower alkyl group; a pyridyl methyl group; a cycloalkyl (with 3 to 6 carbon atoms)-alkyl group; an adamantanemethyl group; a furfuryl group; a cycloalkyl group with 3 to 6 carbon atoms; or an acyl group; and
q is 1 or 2;
(vi) a cyclic amine derivative represented by the following general formula (I-2) or a pharmacologically acceptable salt thereof: 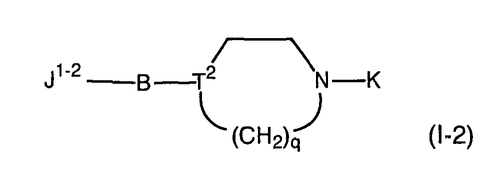
where J¹⁻² is an indanonyl group which may have, as a substituent, a lower alkyl group with 1 to 6 carbon atoms or a lower alkoxy group with 1 to 6 carbon atoms; T² is a nitrogen atom; B, K and q are the same as defined above;
(vii) a cyclic amine derivative selected from the compounds represented by the following formulas or a pharmacologically acceptable salt thereof:

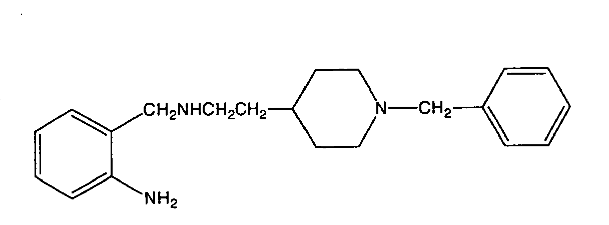

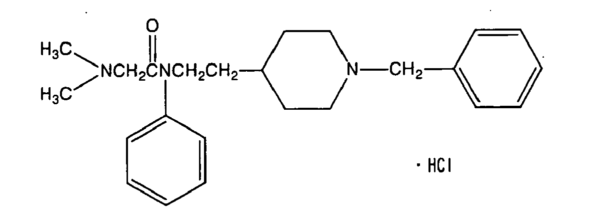

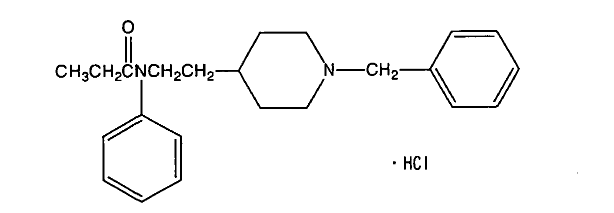

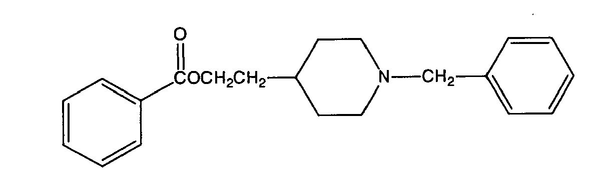

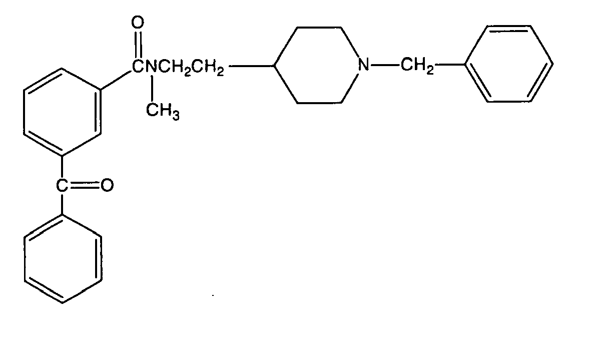

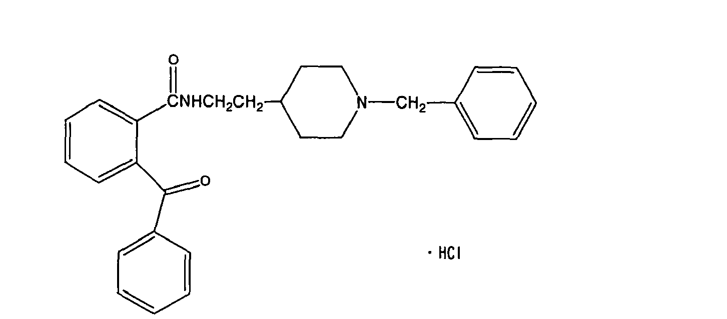

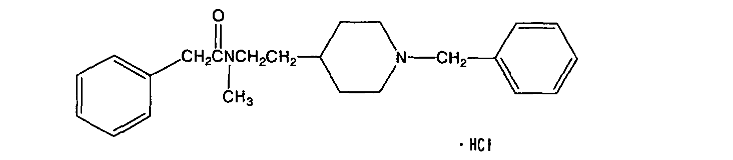

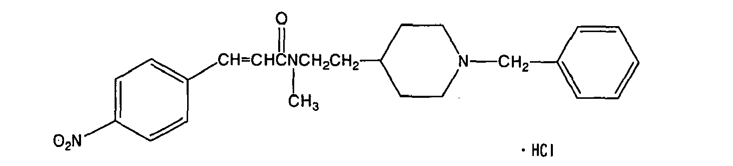

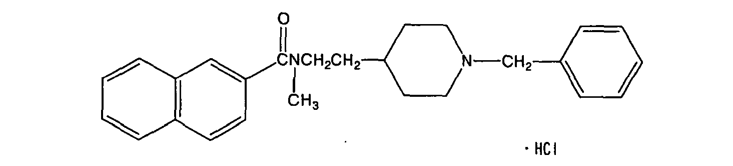

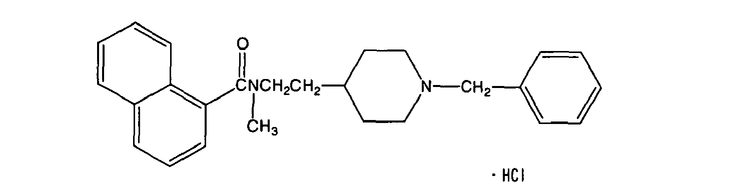

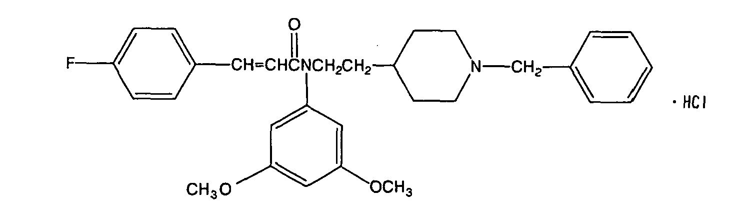

4. The prophylactic and/or therapeutic agent according to claim 3, wherein the salt is a hydrochloride salt.

5. The prophylactic and/or therapeutic agent according to claim 3 or 4, wherein the neurocyte disorder is induced by cerebral ischemia, excitotoxicity or Aβ toxicity.

6. The prophylactic and/or therapeutic agent according to claim 3 or 4, wherein the neurocyte disorder is induced by cerebral ischemia or excitotoxicity associated with any one of cerebral apoplexy, cerebral infarction or cerebral embolism.

7. The prophylactic and/or therapeutic agent according to claim 6, wherein the excitotoxicity is by NMDA or kainic acid.

8. The prophylactic and/or therapeutic agent according to claim 3 or 4, wherein the neurocyte disorder is induced by Aβ toxicity associated with Alzheimer's disease or Down's syndrome.

9. The agent according to any one of claims 1 to 8, wherein the neurons are brain-derived, mature neurons.

10. The agent according to claim 9, wherein the neurons are derived from any one of cerebral cortex, septal area or hippocampus.

11. The agent according to claim 9 or 10, wherein the neurons are primary culture cells.

12. A prognosis improving agent for any disease selected from cerebral apoplexy, cerebral infarction or cerebral embolism, comprising the protective agent according to claim 1 or 2 or the prophylactic and/or therapeutic agent according to claim 3 or 4.

13. A method of protecting neurons of the central nervous system, comprising administering to a patient an effective amount of the protective agent according to claim 1 or 2.

14. A method of preventing and/or treating disorders in neurons of the central nervous system, comprising administering to a patient an effective amount of the prophylactic and/or therapeutic agent according to claim 3 or 4.

15. The method according to claim 14, wherein the disorder in neurons of the central nervous system is induced by cerebral ischemia, excitotoxicity or Aβ toxicity.

16. The method according to claim 15, wherein the excitotoxicity is induced by NMDA or kainic acid.

17. The method according to claim 14, wherein the disorder in neurons of the central nervous system is induced by cerebral ischemia or excitotoxicity associated with any one of cerebral apoplexy, cerebral infarction or cerebral embolism.

18. The method according to claim 14, wherein the disorder in neurons of the central nervous system is induced by Aβ toxicity associated with Alzheimer's disease or Down's syndrome.

19. A method of improving the prognosis of any one of cerebral apoplexy, cerebral infarction or cerebral embolism, comprising administering to a patient an effective amount of the prognosis improving agent according to claim 12.

20. Use of any one of the compounds shown in claim 1 or 2 for preparing any agent selected from the group consisting of the protective agent according to claim 1 or 2, the prophylactic and/or therapeutic agent according to any one of claims 3 to 8, and the prognosis improving agent according to claim 12.

21. A method of screening for a compound with Aβ aggregation inhibitory effect or a pharmacologically acceptable salt thereof, comprising contacting cholinergic neurons of the central nervous system with a candidate compound in the presence of Aβ and detecting or measuring the amount of Aβ aggregation.

22. The method according to claim 21, wherein the results of detection or measurement of the amount of Aβ aggregation are compared with the amount of Aβ aggregation in the absence of the candidate compound to thereby judge whether or not the candidate compound has Aβ aggregation inhibitory effect.

23. A screening kit for a compound with Aβ aggregation inhibitory effect or a pharmacologically acceptable salt thereof, which is for use in the method according to claim 21 or 22.

24. A method for screening for a compound, or a pharmacologically acceptable salt thereof, effective for preventing and/or treating disorders in neurons of the central nervous system induced by Aβ toxicity, comprising contacting cholinergic neurons of the central nervous system with a candidate compound in the presence of Aβ and detecting cytotoxicity or cell death.

25. The method according to claim 24, wherein the results of detection of cytotoxicity or cell death are compared with the extent of cytotoxicity or cell death in the absence of the candidate compound to thereby judge whether or not the candidate compound has cell protective effect against Aβ toxicity.

26. The method according to claim 24 or 25, wherein the detection of cytotoxicity or cell death is performed by measuring the concentration of lactate dehydrogenase or by MTT assay.

27. A screening kit for a compound, or a pharmacologically acceptable salt thereof, effective for preventing and/or treating disorders in neurons of the central nervous system induced by Aβ toxicity, which is for use in the method according to claim 24 or 25.
